# EUROPÄISCHE PATENTSCHRIFT

(11) **EP 4 032 521 B1**
(45) Veröffentlichungstag und Bekanntmachung des Hinweises auf die Patenterteilung: **01.03.2023**
(21) Anmeldenummer: 21152826.0
(22) Anmeldetag: 21.01.2021
(51) Int. Cl.: A61K 6/61, A61K 6/887, A61C 5/70, A61C 13/087

(54) **DENTALWERKSTOFF ZUR HERSTELLUNG PROVISORISCHER KRONEN UND BRÜCKEN**
DENTAL MATERIAL FOR THE PRODUCTION OF TEMPORARY CROWNS AND BRIDGES
MATIÈRE DENTAIRE DESTINÉE À LA FABRICATION DE COURONNES ET PONTS TEMPORAIRES

(43) Veröffentlichungstag der Anmeldung: 27.07.2022
(73) Patentinhaber: Ivoclar Vivadent AG, 9494 Schaan (LI)
(72) Erfinder: Angermann, Jörg, 7320 Sargans (CH); Schädlich, Johannes, 9430 St. Margrethen (CH); Köhler, Thomas, 78479 Reichenau (DE)
(74) Vertreter: Uexküll & Stolberg

(56) Entgegenhaltungen:
- EP-A1- 3 692 976
- EP-B1- 3 692 976
- WO-A1-2007/016508

## Beschreibung

Die vorliegende Erfindung betrifft radikalisch polymerisierbare Zusammensetzungen mit verbessertem Abbindeverhalten, die sich besonders als Dentalwerkstoffe eigenen, insbesondere als Prothesenmaterialien zur Herstellung provisorischer Kronen und Brücken. Die Zusammensetzungen enthalten als Initiator für die radikalische Polymerisation ein Redoxsystem, das ein Hydroperoxid und mindestens drei unterschiedliche Thioharnstoffderivate enthält.

Zur Herstellung von Dentalrestaurationen und insbesondere zur Herstellung temporärer Restaurationen wie z.B. provisorischen Kronen und Brücken werden regelmäßig polymerisierbare Zusammensetzungen auf der Basis von radikalisch polymerisierbaren Monomeren eingesetzt. Als Monomere werden meistens Mischungen von mono- und multifunktionellen (Meth)acrylaten verwendet. Häufig verwendete Dimethacrylate sind 2,2-Bis[4-(2-hydroxy-3-methacryloyloxypropyl)phenyl]propan (Bis-GMA) und 1,6-Bis-[2-methacryloyloxyethoxy-carbonylamino]-2,2,4-trimethylhexan (UDMA), die eine hohe Viskosität haben und Polymerisate mit sehr guten mechanischen Eigenschaften ergeben. Diese werden mit niedrigviskosen Monomeren wie Triethylenglycoldimethacrylat (TEGDMA), 1,10-Decandioldimethacrylat (D3MA) und Bis(3-methacryloyloxymethyl)tricyclo-[5.2.1.02.6]decan (DCP) verdünnt. Zur Härtung werden geeignete Initiatoren zugesetzt, wobei je nach Anwendungsgebiet Photoinitiatoren, thermische Initiatoren, Redox-Initiatorsysteme oder auch Kombinationen davon eingesetzt werden. Materialien zur Herstellung provisorischer Kronen und Brücken enthalten meist Redoxsysteme.

Um eine ausreichende Lagerstabilität der Materialien zu gewährleisten, werden Werkstoffe auf der Basis von Redoxinitiatoren meist als sogenannte Zwei-Komponenten-Systeme (2K-Systeme) eingesetzt, wobei das Oxydationsmittel (Peroxid- oder Hydroperoxid) und das Reduktionsmittel (Amine, Sulfinsäuren, Barbiturate, Thioharnstoff etc.) jeweils in getrennte Komponenten eingearbeitet werden. Diese Komponenten werden kurz vor der Anwendung miteinander gemischt. Die Komponenten müssen so abgestimmt sein, dass sie leicht homogen miteinander gemischt werden können und gut handhabbar sind. Außerdem muss eine für dentale Zwecke ausreichende Verarbeitungszeit gegeben sein. Unter der Verarbeitungszeit wird die Zeitspanne zwischen dem Vermischen der Komponenten und der beginnenden Härtung des gemischten Materials verstanden.

Zur Herstellung von provisorischen Kronen oder Brücken wird ein Abdruck des oder der zu behandelnden Zähne angefertigt. Anschließend werden der oder die Zähne beschliffen, der Abdruck dann mit einem selbsthärtenden Restaurationsmaterial gefüllt und auf die präparierten Zähne gedrückt. Nach dem Härten wird das Material aus dem Abdruck entnommen und durch Beschneiden mit einem Skalpell oder einer Schere oder durch Fräsen nachbearbeitet. Dies gelingt dann am besten, wenn das Material noch elastisch ist. Ist die Härtung zu weit fortgeschritten, ist das Material hart und spröde, was die Nachbearbeitung erschwert und zum Bruch des Provisoriums und zu scharfen Kanten führen kann. Materialien zur Herstellung provisorischer Restaurationen sollen daher bei der Härtung eine möglichst lange elastische Phase aufweisen.

Selbsthärtende Restaurationsmaterialien enthalten oft Redoxinitiatorsysteme, die auf einer Mischung von Dibenzoylperoxid (DBPO) mit tertiären aromatischen Aminen, wie z.B. N,N-Diethanol-p-toluidin (DEPT), N,N-Dimethyl-sym.-xylidin (DMSX) oder N,N-Diethyl-3,5-di-tert.-butylanilin (DABA) beruhen. Mit solchen Redoxinitiatorsystemen lassen sich in Kombination mit phenolischen Inhibitoren die Verarbeitungs- und Aushärtezeit relativ gut einstellen. Ein Nachteil von DBPO/Amin-Systemen sind Verfärbungen, die durch eine langsame Oxidation der Amine verursacht werden.

Hydroperoxid-Redoxinitiatorsysteme weisen diesen Nachteil nicht auf, weil keine Amine als Reduktionsmittel erforderlich sind. Außerdem sind Hydroperoxide thermisch stabiler als Peroxide. Die DE 26 35 595 C2 offenbart polymerisierbare Zahnfüllmassen, die als Initiatorsystem ein substituiertes Thioharnstoff-Reduktionsmittel in Kombination mit einem Hydroperoxid als Oxydationsmittel enthalten. Die Materialien sollen eine verbesserte Farbbeständigkeit, eine hervorragende Härtegeschwindigkeit und eine verbesserte Lagerfähigkeit aufweisen.

Die EP 1 693 046 B1 offenbart dentale Zemente und Stumpfaufbaumaterialien, die ein (2-Pyridyl)-2-thioharnstoffderivat in Kombination mit einem Hydroperoxid enthalten, in dem die Hydroperoxidgruppe an ein tertiäres Kohlenstoffatom gebunden ist.

Die WO 2007/016508 A1 offenbart polymerisierbare dentale Zusammensetzungen, die als Initiatorsystem ein Hydroperoxid in Kombination mit einem Thioharnstoffderivat und 2-Mercapto-1-methylimidazol enthalten. Die Materialien zeichnen sich dadurch aus, dass sie keine säuregruppenhaltigen Komponenten enthalten.

Gemäß EP 1 754 465 B1 soll das Cumolhydroperoxid/Acetylthioharnstoff-System eine unbrauchbar langsame Aushärtungskinetik aufweisen. Zur Überwindung dieses Problems wird die Zugabe von löslichen Kupferverbindungen vorgeschlagen.

Die US 7,275,932 B2 schlägt die Verwendung von Hydroperoxiden und Thioharnstoffderivaten in Kombination mit einer sauren Verbindung als Beschleuniger vor. Bevorzugte saure Verbindungen sind Acrylate und Methacrylate mit Säuregruppen wie z.B. Methacrylsäure.

Die EP 2 233 544 A1 und die EP 2 258 336 A1 offenbaren Dentalwerkstoffe, die ein Hydroperoxid und ein Thioharnstoffderivat in Kombination mit einer Vanadiumverbindung als Beschleuniger enthalten.

Die US 6,815,470 B2 schlägt zur Vermeidung der mit organischen Peroxiden und tertiären Aminen verbundenen Nachteile die Verwendung eines Arylborats in Kombination mit einer sauren Verbindung und einem Peroxid als Initiatorsystem vor. Das Arylborat soll durch Reaktion mit der sauren Verbindung ein Arylboran bilden, das bei der Reaktion mit Sauerstoff polymerisierbare Radikale freisetzt. Als saure Verbindung können polymerisierbare Monomere verwendet werden, die Säuregruppen aufweisen.

Die EP 3 692 975 A1 offenbart radikalisch polymerisierbare Dentalwerkstoffe, die als Initiatorsystem für die radikalische Polymerisation eine Kombination aus einem Thioharnstoffderivat, einem Hydroperoxid und einem Peroxid enthalten. Durch den Zusatz einer geringer Mengen eines Peroxids kann die Reaktivität eines Initiatorsystems auf der Basis eines Hydroperoxids und eines Thioharnstoffderivats deutlich beschleunigt werden.

Die EP 3 692 974 A1 offenbart radikalisch polymerisierbare Dentalwerkstoffe mit einem Initiatorsystem für die radikalische Polymerisation, das neben einem Thioharnstoffderivat, einem Hydroperoxid und einem Peroxid zusätzlich mindestens eine Übergangsmetallverbindung enthält. Durch die Zugabe der Übergangsmetallverbindung können die mechanischen Eigenschaften der Werkstoffe nach der Härtung erheblich verbessert werden.

Aus der EP 3 692 976 A1 sind geruchsarme Hydroperoxide bekannt, die sich in Kombination mit einem Thioharnstoffderivat als Initiatoren für radikalisch polymerisierbare Dentalwerkstoffe eigenen.

Der Erfindung liegt die Aufgabe zugrunde, Dentalmaterialien zur Verfügung zu stellen, die eine für dental Zwecke geeignete Verarbeitungszeit und eine hohe Lagerstabilität aufweisen, die bei der Härtung eine lange elastische Phase durchlaufen und die nach der vollständigen Härtung gute mechanische Eigenschaften haben. Die Materialien sollen sich insbesondere zur Herstellung provisorischer Dentalrestaurationen wie Kronen und Brücken eignen.

Erfindungsgemäß wird diese Aufgabe durch radikalisch polymerisierbare Dentalwerkstoffe gelöst, die als Initiatorsystem ein Hydroperoxid in Kombination mit mindestens drei unterschiedlichen Thioharnstoffderivaten enthalten, nämlich mehrere cyclische und ein oder mehrere acyclische Thioharnstoffderivate.

Die erfindungsgemäßen Dentalwerkstoffe enthalten vorzugsweise mindestens zwei cyclische Thioharnstoffderivate und mindestens ein acyclisches Thioharnstoffderivat. Es wurde gefunden, dass die erfindungsgemäß verwendeten cyclischen Thioharnstoffderivate eine relativ hohe Reaktivität aufweisen und einen schnellen Start der Polymerisation bewirken. Allerdings bricht die Polymerisation schnell ab. Acyclische Thioharnstoffderivate reagieren demgegenüber langsamer, es hat sich aber gezeigt, dass sie länger aktiv sind. Durch die Kombination von schnell reagierenden mit langsam reagierenden Thioharnstoffderivaten kann einerseits ein schneller Reaktionsstart bewirkt und andererseits sichergestellt werden, dass die Materialien vollständig durchhärten und nach der Härtung gute mechanische Eigenschaften aufweisen.

Der rasche Reaktionsstart ermöglicht es, das Material schnell aus dem Mund des Patienten zu entfernen und so die Belastung für den Patienten gering zu halten. Da der Abdrucklöffel während der Härtung mit der Hand gehalten werden muss, wird auch der Aufwand für das behandelnde Personal deutlich reduziert. Nach dem Abklingen der durch das cyclische Thioharnstoffderivat ausgelösten Polymerisation ist das Material nicht vollständig durchgehärtet und befindet sich in einem elastischen Zustand, der eine einfache Nachbearbeitung z.B. durch Beschneiden oder Beschleifen ermöglicht. Da die durch das acyclische Thioharnstoffderivat initiierte Endhärtung sehr viel langsamer verläuft, ist die elastische Phase im Vergleich zu üblichen Werkstoffen deutlich verlängert, was die Nachbearbeitung der Restauration sehr erleichtert.

Es wurde nicht nur gefunden, dass durch die Kombination von unterschiedlich reaktiven Thioharnstoffderivaten eine lange elastische Phase erhalten wird. Besonders erstaunlich war, dass durch die Zugabe eines dritten Thioharnstoffderivate die Lagerstabilität erheblich verbessert werden kann.

Unter cyclischen Thioharnstoffderivaten werden hier solche Verbindungen verstanden, bei denen die Stickstoffatome der Thioharnstoffgruppe zusammen mit dem dazwischen liegenden Kohlenstoffatom und weiteren Kohlenstoffatomen ein heterocyclisches Ringsystem bilden. Bevorzugt sind cyclische Thioharnstoffderivate der Formel (I): 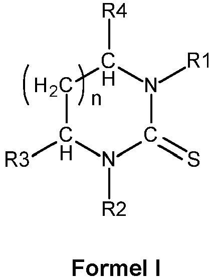 in der:
- R¹, R²: jeweils H oder ein C₁-C₄₋Alkylrest sind, wobei mindestens einer dieser Reste H ist;
- R³, R⁴: unabhängig voneinander jeweils H, ein C₁-C₄₋Alkylrest oder ein C₁-C₄₋Alkoxyrest sind oder zusammen mit den Kohlenstoffatomen, an die sie gebunden sind, und dem dazwischen liegenden Kohlenstoffatom einen sechsgliedrigen, carbocyclischen, aliphatischen oder aromatischen Ring bilden, der durch einen oder mehrere, vorzugsweise 1 oder 2, C₁-C₄-Alkylreste und/oder C₁-C₄-Alkoxyreste substituiert sein kann;
- n: 0, 1, 2 oder 3, vorzugsweise 0 oder 1 ist.

Vorzugsweise haben die Variablen der Formel I die folgenden Bedeutungen:
- R¹, R²: jeweils H oder ein C₁-C₂-Alkylrest, vorzugsweise H oder Methyl, wobei mindestens einer dieser Reste H ist;
- R³, R⁴: jeweils H, ein C₁-C₂-Alkylrest, vorzugsweise Methyl, ein C₁-C₂-Alkoxyrest, vorzugsweise Methoxy, oder bilden zusammen mit den Kohlenstoffatomen, an die sie gebunden sind, und dem dazwischen liegenden Kohlenstoffatom einen Benzolring, der durch einen C₁-C₂-Alkylrest, vorzugweise Methyl, oder einen C₁-C₂-Alkoxyrest, vorzugsweise Methoxy, substituiert sein kann;
- n: 0 oder 1.

Besonders bevorzugt haben die Variablen der Formel I die folgenden Bedeutungen:
- R¹, R²: jeweils H oder Methyl, wobei mindestens einer dieser Reste H ist;
- R³, R⁴: jeweils H, ein C₁-C₂-Alkylrest, vorzugsweise Methyl, oder ein C₁-C₂-Alkoxyrest, vorzugsweise Methoxy, und
- n: 1, oder
- R¹, R²: jeweils H oder Methyl, wobei mindestens einer dieser Reste H ist;
- R³, R⁴: bilden zusammen mit den Kohlenstoffatomen, an die sie gebunden sind, und dem dazwischen liegenden Kohlenstoffatom einen Benzolring, der durch einen C₁-C₂-Alkylrest, vorzugweise Methyl, oder einen C₁-C₂-Alkoxyrest, vorzugsweise Methoxy, substituiert sein kann und
- n: 0.

In allen Fällen umfasst die Formel I auch die entsprechenden Isothioharnstoffderivate.

Besonders bevorzugt sind 3,4,5,6-Tetrahydro-2-pyrimidinthiol (1), 2-Imidazolidinthion (2), 2-Mercaptobenzimidazol (4), 1-Methyl-1H-benzimidazol-2-thiol und 2-Mercapto-5-methoxy-benzimidazol.

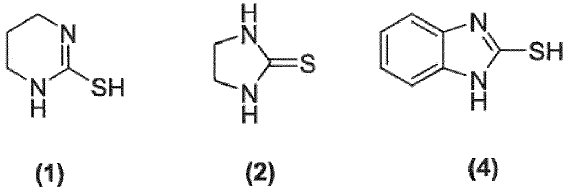

Unter acyclischen Thioharnstoffderivaten werden hier solche Verbindungen verstanden, bei denen die Stickstoffatome der Thioharnstoffgruppe nicht in ein Ringsystem eingebunden sind. Acyclische Thioharnstoffderivate können aber dennoch cyclische Gruppen enthalten.

Bevorzugt sind acyclische Thioharnstoffderivate der Formel (II) 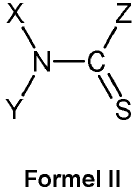 in der
- X: H oder Y ist,
- Y: ein Alkylrest mit 1 bis 8 Kohlenstoffatomen, ein Cycloalkylrest mit 5 oder 6 Kohlenstoffatomen, ein Chlor-, Hydroxy- oder Mercapto-substituierter Alkylrest mit 1 bis 8 Kohlenstoffatomen, ein Alkenylrest mit 3 bis 4 Kohlenstoffatomen, ein Arylrest mit 6 bis 8 Kohlenstoffatomen, ein Chlor-, Hydroxy-, Methoxy- oder Sulfonyl-substituierter Phenylrest, ein Acylrest mit 2 bis 8 Kohlenstoffatomen, ein Chlor- oder Methoxysubstituierter Acylrest, ein Aralkylrest mit 7 bis 8 Kohlenstoffatomen oder ein Chlor- oder Methoxy-substituierter Aralkylrest ist, und
- Z: NH₂, NHX oder NX₂ ist.

Weiter bevorzugte Thioharnstoffderivate sind die in der EP 1 754 465 A1 in Absatz [0009] aufgeführten Verbindungen.

Besonders bevorzugt sind acyclische Thioharnstoffderivate der Formel (III):

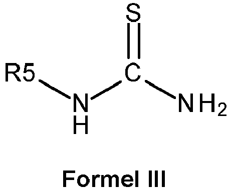

In der
- R⁵: ein C₁-C₁₂-Alkylrest, vorzugsweise ein C₂-C₁₀-Alkylrest, besonders bevorzugt ein C₃-C₈-Alkylrest und ganz besonders bevorzugt ein C₄-C₆-Alkylrest, ein C₁-C₁₂-Alkenrest, vorzugsweise ein C₂-C₁₀-Alkenrest, besonders bevorzugt ein C₃-C₈-Alkenrest und ganz besonders bevorzugt ein C₄-C₆-Alkenrest, ein C₁-C₁₂-Acylrest, vorzugsweise ein C₂-C₁₀-Acylrest, besonders bevorzugt ein C₃-C₈-Acylrest und ganz besonders bevorzugt ein C₄-C₆-Acylrest, ein Pyridyl- oder Phenylrest ist.

Ganz besonders bevorzugte acyclische Thioharnstoffderivate sind Acetyl-, Allyl-, Pyridyl- und Phenylthioharnstoff, Hexanoylthioharnstoff (3) und Mischungen davon. Am meisten bevorzugt ist Hexanoylthioharnstoff (3).

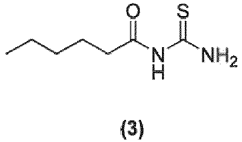

Die erfindungsgemäßen Zusammensetzungen enthalten mindestens 3, vorzugsweise 3 bis 6, besonders bevorzugt 3 oder 4 und ganz besonders bevorzugt 3 unterschiedliche Thioharnstoffderivate. Die Zusammensetzungen enthalten mehrere cylische und ein oder mehrere acyclische Thioharnstoffderivate. Bevorzugt sind Zusammensetzungen die zwei bis vier, vorzugsweise zwei cyclische und ein acyclisches Thioharnstoffdrivat enthalten.

Bevorzugt sind Kombinationen die als acyclisches Thioharnstoffderivat Hexanoylthioharnstoff und mindestens zwei, vorzugsweise genau zwei, cyclische Thioharnstoffderivate enthalten. Besonders bevorzugt sind Zusammensetzungen, die Hexanoylthioharnstoff, 2-Mercaptobenzimidazol oder 2-Imidazolinthion und mindestens ein, vorzugsweise genau ein (1), weiteres cyclische Thioharnstoffderivat enthalten. Ganz besonders bevorzugt sind Zusammensetzungen, die Hexanoylthioharnstoff, 2-Mercaptobenzimidazol oder 2-Imidazolinthion und 3,4,5,6-Tetrahydro-2-pyrimidinthiol enthalten. Eine besonders bevorzugte Kombination von drei Thioharnstoffderivaten ist eine Mischung von 3,4,5,6-Tetrahydro-2-pyrimidinthiol (1), 2-Imidazolidinthion (2) und Hexanoylthioharnstoff (3).

Erfindungsgemäß bevorzugte Hydroperoxide sind Verbindungen der Formel R⁶-(OOH)ₘ, in der R⁶ ein aliphatischer oder aromatischer Kohlenwasserstoffrest und m 1 oder 2 ist. Bevorzugte Reste R sind Alkyl- und Arylgruppen. Die Alkylgruppen können geradkettig, verzweigt oder cyclisch sein. Cyclische Alkylreste können durch aliphatische Alkylgruppen substituiert sein. Bevorzugt sind Alkylgruppen mit 4 bis 10 Kohlenstoffatomen. Arylgruppen können unsubstituiert oder durch Alkylgruppen substituiert sein. Bevorzugte aromatische Kohlenwasserstoffreste sind Benzolreste, die mit 1 oder 2 Alkylgruppen substituiert sind. Die aromatischen Kohlenwasserstoffreste enthalten vorzugsweise 6 bis 12 Kohlenstoffatome. Besonders bevorzugte Hydroperoxide sind t-Amylhydroperoxid, 1,1,3,3-Tetramethylbutylhydroperoxid, t-Butylhydroperoxid, t-Hexylhydroperoxid, 2,5-Dimethyl-2,5-di(hydroperoxy)hexan, Diisopropylbenzolmonohydroperoxid (DIHP), Paramenthanhydroperoxid, p-Isopropylcumolhydroperoxid und Mischungen davon. Ganz besonders bevorzugt sind DIHP und Cumolhydroperoxid (CHP).

Außerdem sind erfindungsgemäß Hydroperoxide gemäß der folgenden Formel (IV) bevorzugt, 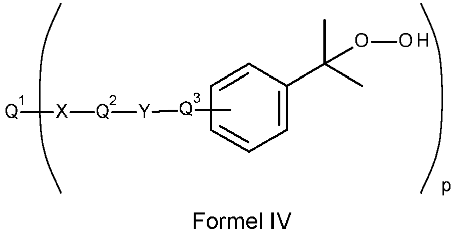 in der die Variablen die folgenden Bedeutungen haben:
- Q¹: ein p-wertiger, aromatischer, aliphatischer, linearer oder verzweigter C₁-C₁₄-Kohlenwasserstoffrest, der durch ein oder mehrere S- und/oder O-Atome unterbro-chen sein kann und der unsubstituiert oder durch einen oder mehrere Substituenten, die vorzugsweise aus -OH, -OR⁷, -Cl und -Br ausgewählt sind, substituiert sein kann, wobei R⁷ ein aliphatischer, linearer oder verzweigter C₁-C₁₀-Kohlenwassrstoffrest ist,
- X, Y: unabhängig voneinander jeweils entfällt, -O-, -COO-; -CONR⁸-, oder -O-CO-NR⁹-, wobei R⁸ und R⁹ unabhängig voneinander für H oder einen C₁-C₅-Alkylrest, vorzugsweise für H, Methyl und/oder Ethyl, besonders bevorzugt H stehen, und wobei X und Y vorzugsweise nicht gleichzeitig entfallen,
- Q²: entfällt, ein aliphatischer, linearer oder verzweigter C₁-C₁₄-Alkylen-Rest, der durch S- und/oder O-Atome unterbrochen sein kann und der unsubstituiert oder durch -OH, -OR¹⁰, -CI und/oder -Br substituiert sein kann, wobei R¹⁰ ein aliphatischer, linearer oder verzweigter C₁-C₁₀-Kohlenwasserstoffrest ist,
- Q³: eine C₁-C₃₋Alkylengruppe oder entfällt, vorzugsweise -CH₂- oder entfällt, wobei X und/oder Y entfällt, wenn Q² entfällt,
- p: 1, 2, 3 oder 4, und wobei die Substitution am Aromaten in 2-, 3- oder 4-Stellung erfolgt, relativ zur Cumolhydroperoxidgruppe.

Vorzugsweise haben die Variablen die folgenden Bedeutungen:
- Q¹: ein 1- oder 2-wertiger, aliphatischer, linearer oder verzweigter C₁-C₁₀-Kohlenwasser-stoffrest, der durch ein oder mehrere O-Atome, vorzugsweise ein O-Atom, unterbrochen sein kann und der durch einen oder mehrere, vorzugsweise einen, Substituenten, die aus -OH und -OR⁷ ausgewählt sind, substituiert sein kann oder vorzugsweise unsubstituiert ist, wobei R⁷ ein aliphatischer, linearer oder verzweigter C₁-C₆-Kohlenwassrstoffrest ist,
- X, Y: unabhängig voneinander jeweils entfällt, -O-, -COO- oder -O-CO-NR⁹-, wobei R⁹ für H oder einen C₁-C₅-Alkylrest, vorzugsweise für H, Methyl und/oder Ethyl und ganz besonders bevorzugt für H steht, und wobei X und Y vorzugsweise nicht gleichzeitig entfallen,
- Q²: entfällt, ein linearer oder verzweigter C₁-C₁₀-Alkylen-Rest, der durch ein oder mehrere O-Atome, vorzugsweise ein O-Atom, unterbrochen sein kann und der durch einen oder mehrere, vorzugsweise einen, Substituenten, die aus -OH und -OR¹⁰ ausgewählt sind, substituiert sein kann oder vorzugsweise unsubstituiert ist, wobei R¹⁰ ein aliphatischer, linearer oder verzweigter C₁-C₆-Kohlenwassrstoffrest ist,
- p: 1 oder 2, und wobei die Substitution am Aromaten in 3-, vorzugsweise in 4-Stellung erfolgt.

Besonders bevorzugt haben die Variablen die folgenden Bedeutungen:
- Q¹: ein 1- oder 2-wertiger, aliphatischer, linearer oder verzweigter C₁-C₅-Kohlenwasser-stoffrest, der durch ein O-Atom unterbrochen sein kann und der durch eine OH-Gruppe substituiert sein kann,
- X: -COO-,
- Y: entfällt,
- Q²: entfällt oder ein linearer C₁-C₃-Alkylen-Rest,
- p: 1 oder 2, und wobei die Substitution am Aromaten in 4-Stellung erfolgt.

Ganz besonders bevorzugt haben die Variablen die folgenden Bedeutungen:
- Q¹: ein 1- oder 2-wertiger, aliphatischer, verzweigter, vorzugsweise linearer C₁-C₄-Kohlenwasserstoffrest,
- X: -COO-,
- Y: entfällt,
- Q²: entfällt oder ein Methylen-Rest,
- p: 1 oder 2, und wobei die Substitution am Aromaten in 4-Stellung erfolgt.

Hydroperoxide der Formel (IV) und deren Herstellung werden in EP 3 692 976 A1 genauer beschrieben. Sie zeichnen sich insbesondere dadurch aus, dass sie keinen unangenehmen Geruch aufweisen. Ein ganz besonders bevorzugtes Hydroperoxid der Formel (IV) ist 4-(2-Hydroperoxypropan-2-yl)phenylpropionat (K220): 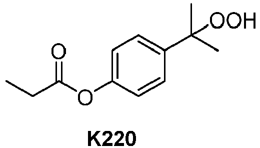 Weiter bevorzugt sind die in Absatz [0025] der EP 3 692 976 A1 konkret genannten Hydroperoxide.

Gemäß einer bevorzugten Ausführungsform enthalten die erfindungsgemäßen Werkstoffe zusätzlich eine Übergangsmetallverbindung.

Erfindungsgemäß bevorzugte Übergangsmetallverbindungen sind Verbindungen, die sich von Übergangsmetallen ableiten, die mindestens zwei stabile Oxidationsstufen haben. Besonders bevorzugt sind Verbindungen der Elemente Kupfer, Eisen, Kobalt, Nickel und Mangan. Diese Metalle weisen die folgenden stabilen Oxydationsstufen auf: Cu(I)/Cu(II), Fe(II)/Fe(III), Co(II)/Co(III), Ni(II)/Ni(III), Mn(II)/Mn(III). Werkstoffe, die mindestens eine Kupfer-Verbindung enthalten, sind besonders bevorzugt.

Die Übergangsmetalle werden bevorzugt in Form ihrer Salze eingesetzt. Bevorzugte Salze sind die Nitrate, Acetate, 2-Ethyl-hexanoate und Halogenide, wobei Chloride besonders bevorzugt sind.

Die Übergangsmetalle können weiterhin vorteilhaft in komplexierter Form eingesetzt werden, wobei Komplexe mit chelatbildenden Liganden besonders bevorzugt sind. Bevorzugte einfache Liganden zur Komplexierung der Übergangsmetalle sind 2-Ethylhexanoat und THF. Bevorzugte chelatbildende Liganden sind 2-(2-Aminoethylamino)ethanol, aliphatische Amine, besonders bevorzugt 1,1,4,7,10,10-Hexamethyltriethylentetramin (HMTETA), N,N,N',N",N"-Pentamethyldiethylentriamin (PMDETA), Tris[2-(dimethylamino)ethyl]amin (Me₆TREN), N,N,N',N'-Tetramethylethylendiamin (TMEDA), 1,4,8,11-Tetraaza-1,4,8,11-tetramethylcyclo-tetradecan (Me4CYCLAM), Diethylentriamin (DETA), Triethylentetramin (TETA) und 1,4,8,11-Tetraazacyclotetradecan (CYCLAM)); pyridinhaltige Liganden, besonders bevorzugt N,N,N',N'-Tetrakis(2-pyridylmethyl)ethylendiamin (TPEN), N,N-Bis(2-pyridylmethyl)amin (BPMA), N,N-Bis(2-pyridylmethyl)octylamin (BPMOA), 2,2'-Bipyridin und 8-Hydroxychinolin. Ganz besonders bevorzugte Liganden sind Acetylaceton, Dimethylglyoxim und 1,10-Phenanthrolin.

Bei elektrisch neutralen Liganden muss die Ladung der Übergangsmetallionen durch geeignete Gegenionen ausgeglichen werden. Hierzu kommen insbesondere die oben genannten Ionen in Betracht, die zur Bildung von Salzen verwendet werden, wobei Acetate und Chloride besonders bevorzugt sind. Chloride und Komplexe zeichnen sich durch eine relativ gute Löslichkeit in Monomeren aus, die zur Herstellung von Dentalwerkstoffen eingesetzt werden.

Anstelle der Übergangsmetallkomplexe können nichtkomplexe Salze der Übergangsmetalle in Kombination mit komplexbildenden organischen Verbindungen zur Herstellung der Dentalwerkstoffe eingesetzt werden, vorzugsweise in Kombination mit den oben genannten chelatbildenden Verbindungen. Die organischen Liganden bilden beim Mischen mit den Übergangsmetallsalzen die katalytisch wirksamen Komplexe. Die Verwendung solcher Kombinationen von Übergangsmetallsalzen und organischen Liganden ist bevorzugt.

Bevorzugt sind Übergangsmetallverbindungen der Metalle Kupfer, Eisen, Kobalt und Nickel.

Bevorzugte Kupfersalze sind Cu(II)-carboxylate (z.B. der Essigsäure oder 2-Ethylhexansäure), CuCl₂, CuBr₂, CuI₂, besonders bevorzugt CuBr und ganz besonders bevorzugt CuCl. Bevorzugte Kupferkomplexe sind Komplexe mit den Liganden Acetylaceton, Phenanthrolin (z.B. 1,10-Phenanthrolin (Phen)), der aliphatischen Aminen, wie z.B. 1,1,4,7,10,10-Hexamethyltriethylentetramin (HMTETA), N,N,N',N",N"-Pentamethyldiethylen-triamin (PMDETA), Tris[2-(dimethylamino)ethyl]amin (Me₆TREN).

Bevorzugte Eisensalze sind FeCl₃, FeBr₂ und FeCl₂. Bevorzugte Eisenkomplexe sind Komplexe mit den Liganden Acetylaceton, Triphenylphosphin, 4,4'-Di(5-nonyl)-2,2'-bipyridin (dNbpy) oder 1,3-Diisopropyl-4,5-dimethylimidazol-2-yliden (Prilm). Ganz besonders bevorzugt sind die Komplexe Fe(acac)₂ und FeCl₂(PPh₃)₂.

Bevorzugte Nickelsalze sind NiBr₂ und NiCl₂, bevorzugte Nickelkomplexe sind Nickelacetylacetonat und NiBr₂(PPh₃)₂.

Erfindungsgemäß sind Kupferverbindungen, Kupferkomplexe und insbesondere Mischungen von Kupfersalzen und komplexierenden organischen Liganden besonders bevorzugt. Ganz besonders bevorzugt sind Salze und Komplexe des einwertigen Kupfers (Cu⁺), am meisten bevorzugt ist Kupfer(I)chlorid (CuCI). Zusammensetzungen, die ein Salz des einwertigen Kupfers und insbesondere CuCI enthalten, zeichnen sich durch eine besonders gute Lagerstabilität aus.

Das erfindungsgemäße Initiatorsystem eignet sich besonders zur Aushärtung radikalisch polymerisierbarer Zusammensetzungen.

Erfindungsgemäße Zusammensetzungen enthalten neben dem Initiatorsystem vorzugsweise mindestens ein radikalisch polymerisierbares Monomer. Besonders bevorzugt sind Zusammensetzungen, die als radikalisch polymerisierbares Monomer mindestens ein mono- oder multifunktionelles (Meth)acrylat enthalten. Unter monofunktionellen (Meth)acrylaten werden Verbindungen mit einer, unter multifunktionellen (Meth)acrylaten Verbindungen mit zwei oder mehr, vorzugsweise 2 bis 4 radikalisch polymerisierbaren Gruppen verstanden. Gemäß einer ganz besonders bevorzugten Ausführungsform enthalten die erfindungsgemäßen Zusammensetzungen mindestens ein Dimethacrylat oder eine Mischung von Mono- und Dimethacrylaten. Materialien, die intraoral gehärtet werden sollen, enthalten als radikalisch polymerisierbares Monomer vorzugsweise mono- und/oder multifunktionelle Methacrylate.

Bevorzugte mono- oder multifunktionelle (Meth)acrylate sind Methyl-, Ethyl-, 2-Hydroxyethyl-, Butyl-, Benzyl-, Tetrahydrofurfuryl- oder Isobornyl(meth)acrylat, p-Cumylphenoxyethylenglycolmethacrylat (CMP-1E), 2-(2-Biphenyloxy)-ethylmethacrylat, Bisphenol-A-dimethacrylat, Bis-GMA (ein Additionsprodukt aus Methacrylsäure und Bisphenol-A-diglycidylether), ethoxy- oder propoxyliertes Bisphenol-A-Dimethacrylat, wie z.B. 2-[4-(2-Methacryloyloxyethoxyethoxy)phenyl]-2-[4-(2-methacryloyloxyethoxy)phenyl]propan) (SR-348c, Firma Sartomer; enthält 3 Ethoxygruppen) und 2,2-Bis[4-(2-methacryloxy-propoxy)phenyl]propan, UDMA (ein Additionsprodukt aus 2-Hydroxyethylmethacrylat und 2,2,4-Trimethylhexamethylen-1,6-diisocyanat), V-380 (ein Additionsprodukt aus einer Mischung von 0,7 mol 2-Hydroxyethylmethacrylat und 0,3 mol 2-Hydroxypropylmethacrylat mit 1 mol α,α,α',α'-Tetramethyl-m-xylylen-diisocyanat), Di-, Tri- oder Tetraethylenglycoldimethacrylat, Trimethylolpropantrimethacrylat, Pentaerythrittetramethacrylat sowie Glycerindi- und -trimethacrylat, 1,4-Butandioldimethacrylat, 1,10-Decandioldimethacrylat (D₃MA), Bis(methacryloyloxymethyl)tricyclo-[5.2.1.02,6]decan (DCP), Polyethylenglycol- oder Polypropylenglycoldimethacrylate, wie z.B. Polyethylenglycol-200- oder Polyethylenglycol-400-dimethacrylat (PEG-200- oder PEG-400-DMA) oder 1,12-Dodecandioldimethacrylat, oder eine Mischung davon.

Die erfindungsgemäßen Zusammensetzungen können neben dem erfindungsgemäßen Initiatorsystem vorteilhaft zusätzlich einen Initiator für die radikalische Photopolymerisation enthalten. Solche Zusammensetzungen sind dualhärtend, d.h. sie lassen sich sowohl chemisch als auch durch Licht härten. Bevorzugte Photoinitiatoren sind Benzophenon, Benzoin sowie deren Derivate, α-Diketone und deren Derivate, wie 9,10-Phenanthrenchinon, 1-Phenyl-propan-1,2-dion, Diacetyl und 4,4'-Dichlorbenzil. Bevorzugt werden Campherchinon (CC) und 2,2-Dimethoxy-2-phenyl-acetophenon in Kombination mit Aminen als Reduktionsmittel, wie z.B. 4-(Dimethylamino)-benzoesäureethylester (EDMAB), oder N,N-Dimethylaminoethylmethacrylat, verwendet.

Erfindungsgemäß sind solche Zusammensetzungen bevorzugt, die keine Amine enthalten. Besonders bevorzugt sind daher Norrish-Typ-I-Photoinitiatoren. Norrish-Typ-I-Photoinitiatoren benötigen keine Aminkomponente.

Bevorzugte Norrish-Typ-I-Photoinitiatoren sind Acyl- oder Bisacylphosphinoxide. Besonders bevorzugt sind Monoacyltrialkylgermanium-, Diacyldialkylgermanium- und Tetraacylgermanium-Verbindungen, wie z.B. Benzoyltrimethylgerman, Dibenzoyldiethylgerman, Bis(4-methoxybenzoyl)diethylgerman (Ivocerin^{®}), Tetrabenzoylgerman und Tetrakis(o-methyl-benzoyl)german. Außerdem lassen sich Mischungen der verschiedenen Photoinitiatoren einsetzen, wie z.B. Bis(4-methoxybenzoyl)diethylgerman bzw. Tetrakis(o-methylbenzoyl)-german in Kombination mit Campherchinon und 4-Dimethylaminobenzoesäureethylester.

Die erfindungsgemäßen Zusammensetzungen können vorteilhaft außerdem einen oder mehrere organische oder anorganische Füllstoffe enthalten. Bevorzugt sind partikuläre Füllstoffe. Füllstoffhaltige Zusammensetzungen eignen sich besonders als dentale Befestigungszemente oder Füllungskomposite sowie zur Herstellung provisorischer Kronen und Brücken.

Bevorzugte anorganische Füllstoffe sind Oxide, wie SiO₂, ZrO₂ und TiO₂ oder Mischoxide aus SiO₂, ZrO₂, ZnO und/oder TiO₂, nanopartikuläre oder mikrofeine Füllstoffe, wie pyrogene Kieselsäure oder Fällungskieselsäure, Glaspulver, wie Quarz-, Glaskeramik-, Borosilikat- oder röntgenopake Glaspulver, vorzugsweise Barium- oder Strontiumaluminiumsilikatgläser, und röntgenopake Füllstoffe, wie Ytterbiumtrifluorid, Tantal(V)-oxid, Bariumsulfat oder Mischoxide von SiO₂ mit Ytterbium(III)-oxid oder Tantal(V)-oxid. Weiterhin können die erfindungsgemäßen Dentalwerkstoffe faserförmige Füllstoffe, Nanofasern, Whiskers oder Mischungen davon enthalten.

Bevorzugt weisen die Oxide eine Partikelgröße von 0,010 bis 15 µm, die nanopartikulären oder mikrofeinen Füllstoffe eine Partikelgröße von 10 bis 300 nm, die Glaspulver eine Partikelgröße von 0,01 bis 15 µm, vorzugweise von 0,2 bis 1,5 µm, und die röntgenopaken Füllstoffe eine Partikelgröße von 0,2 bis 5 µm auf.

Besonders bevorzugte Füllstoffe sind Mischoxide aus SiO₂ und ZrO₂, mit einer Partikelgröße von 10 bis 300 nm, Glaspulver mit einer Partikelgröße von 0,2 bis 1,5 µm, insbesondere röntgenopake Glaspulver von z.B. Barium- oder Strontiumaluminiumsilikatgläsern, und röntgenopake Füllstoffe mit einer Partikelgröße von 0,2 bis 5 µm, insbesondere Ytterbiumtrifluorid und/oder Mischoxide von SiO₂ mit Ytterbium(III)-oxid.

Außerdem sind als Füllstoff gemahlene Präpolymerisate oder Perlpolymerisate (Isofüller) geeignet. Diese können ausschließliche aus organischen Polymeren oder aus organischen Polymeren bestehen, die ihrerseits mit anorganischen Füllstoffen wie röntgenopakem/n Glaspulver(n) und Ytterbiumtrifluorid gefüllt sind. Zur Herstellung der gemahlenen Präpolymerisate und Perlpolymere eignen sich die oben definierten Monomere und Füllstoffe. Zusammensetzungen zur Herstellung dentaler Vollprothesen enthalten als Füllstoffe vorzugsweise ausschließlich organische Füllstoffe, besonders bevorzugt gemahlene Polymerisate oder Perlpolymerisate auf Basis von Polymethylmethacrylat (PMMA), ganz besonders bevorzugt Perlpolymerisate auf der Basis von PMMA.

Wenn nicht anders angegeben, handelt es sich bei allen Partikelgrößen um gewichtsmittlere Partikelgrößen, wobei die Partikelgrößenbestimmung im Bereich von 0,1 µm bis 1000 µm mittels statischer Lichtstreuung erfolgt, vorzugsweise mit einem statischen Laserstreuungs-Partikelgrößen-Analysator LA-960 (Horiba, Japan). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine 0,1 bis 0,5%ige wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320.

Partikelgrößen kleiner als 0,1 µm werden vorzugsweise mittels Dynamischer Lichtstreuung (DLS) bestimmt. Die Messung der Partikelgröße im Bereich von 5 nm bis 0,1 µm erfolgt vorzugsweise durch Dynamische Lichtstreuung (DLS) von wässrigen Partikeldispersionen, vorzugsweise mit einem Malvern Zetasizer Nano ZS (Malvern Instruments, Malvern UK) mit einem He-Ne-Laser mit einer Wellenlänge von 633 nm, bei einem Streuwinkel von 90° bei 25°C.

Partikelgrößen kleiner als 0,1 µm können auch mittels REM- oder TEM-Spektroskopie bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf einem 50 Å dickem Kupfergitter (Maschenweite 300) aufgebracht, das mit Kohlenstoff beschichtet ist, und im Anschluss das Lösungsmittel verdampft.

Die Lichtstreuung nimmt mit abnehmender Partikelgröße ab, jedoch haben Füllstoffe mit geringer Partikelgröße eine größere Verdickungswirkung. Die Füllstoffe werden nach ihrer Partikelgröße unterteilt in Makrofüller und Mikrofüller, wobei Füllstoffe mit einer mittleren Partikelgröße von 0,2 bis 10 µm als Makrofüller und Füllstoffe mit einer mittlere Partikelgröße von ca. 5 bis 100 nm als Mikrofüller bezeichnet werden. Makrofüller werden z.B. durch Mahlen z.B. von Quarz, röntgenopaken Gläsern, Borosilikaten oder von Keramik gewonnen und bestehen meist aus splitterförmigen Teilen. Mikrofüller wie Mischoxide können z.B. durch hydrolytische Co-Kondensation von Metallalkoxiden hergestellt werden.

Zur Verbesserung des Verbundes zwischen den Füllstoffpartikeln und der vernetzten Polymerisationsmatrix sind die Füllstoffe vorzugsweise oberflächenmodifiziert, besonders bevorzugt durch Silanisierung, ganz besonders bevorzugt durch radikalisch polymerisierbare Silane, insbesondere mit 3-Methacryloyloxypropyltrimethoxysilan. Zur Oberflächenmodifizierung von nicht-silikatischen Füllstoffen, z.B. von ZrO₂ oder TiO₂, können auch funktionalisierte saure Phosphate, wie z.B. 10-Methacryloyloxydecyldihydrogenphosphat eingesetzt werden.

Außerdem können die erfindungsgemäßen Zusammensetzungen ein oder mehrere weitere Additive enthalten, vor allem Stabilisatoren, Farbmittel, mikrobiozide Wirkstoffe, fluoridionenabgebende Additive, Treibmittel, optische Aufheller, Weichmacher und/oder UV-Absorber.

Die erfindungsgemäßen Werkstoffe umfassen vorzugsweise zwei räumlich getrennte Komponenten, die zur Anwendung mit einander gemischt werden. Die erste Komponente (Katalysatorpaste) enthält das oder die Hydroperoxide, und die zweite Komponente (Basenpaste) enthält die Thioharnstoffderivate und ggf. die Übergangsmetallverbindung. Basenpaste und Katalysatorpaste werden vorzugsweise in einem Volumenverhältnis von 1:1 miteinander gemischt. Durch das Mischen von Basen- und Katalysatorpaste wird die Härungsreaktion initiiert.

Die Basenpaste enthält vorzugsweise 0,07 bis 3,50 Gew.-%, besonders bevorzugt 0,07 bis 3,3 Gew.-%, ganz besonders bevorzugt 0,20 bis 2,50 Gew.-% und am meisten bevorzugt 0,35 bis 2,10 Gew.-% mindestens zweier cyclischer Thioharnstoffderivate und 0,04 bis 2,3 Gew.-%, besonders bevorzugt 0,04 bis 2,00 Gew.-%, besonders bevorzugt 0,10 bis 1,5 Gew.-% und am meisten bevorzugt 0,20 bis 1,2 Gew.-% mindestens eines acyclischen Thioharnstoffderivats.

Gemäß einer bevorzugten Ausführungsform enthält die Basenpaste 0,03 bis 2,50 Gew.-%, besonders bevorzugt 0,10 bis 2,00 Gew.-% und am meisten bevorzugt 0,15 bis 1,50Gew.-% 3,4,5,6-Tetrahydro-2-pyrimidinthiol und/oder 0,04 bis 1,30 Gew.-%, besonders bevorzugt 0,10 bis 1,00 Gew.-% und am meisten bevorzugt 0,15 bis 0,60 Gew.-% 2-Mercaptobenzimidazol und/oder 0,04 bis 2,00 Gew.-%, besonders bevorzugt 0,10 bis 1,50 Gew.-% und am meisten bevorzugt 0,25 bis 1,20 Gew.-% Hexanoylthioharnstoff, wobei Materialen die alle drei genannten cyclischen Thioharnstoffderivate jeweils in den aufgeführten Mengen enthalten, besonders vorteilhaft sind.

Die Mengen der einzelnen Thioharnstoffderivate werden dabei vorzugsweise so gewählt, dass das Gewichtsverhältnis von 3,4,5,6-Tetrahydro-2-pyrimidinthiol zu 2-Mercaptobenzimidazol in einem Bereich von 0,15 bis 4,00, besonders bevorzugt 0,20 bis 3,50 und am meisten bevorzugt 0,25 bis 3,00 liegt. Das Gewichtsverhältnis von 3,4,5,6-Tetrahydro-2-pyrimidinthiol zu Hexanoylthioharnstoff liegt vorzugsweise in einem Bereich von 0,04 bis 2,50, besonders bevorzugt 0,40 bis 3,00 und am meisten bevorzugt 0,60 bis 2,70. Das Gewichtsverhältnis von 2-Mercaptobenzimidazol zu Hexanoylthioharnstoff liegt vorzugsweise in einem Bereich von 0,05 bis 2,50, besonders bevorzugt 0,10 bis 1,2 und am meisten bevorzugt 0,20 bis 0,90.

Vorzugsweise enthält die Basenpaste insgesamt 0,10 bis 4,00 Gew.-%, besonders bevorzugt 0,50 bis 2,50 Gew.-% und am meisten bevorzugt 1,00 bis 2,00 Gew.-% an Thioharnstoffderivaten.

Die Thioharnstoffderivate werden vorzugsweise in einer molaren Gesamtmenge von 50 bis 400 mol-%, vorzugsweise 75 bis 300 mol-% und ganz besonders bevorzugt 100 bis 200 mol-%, bezogen auf die molare Menge an Hydroperoxid in der Katalysatorpaste eingesetzt.

Vorzugsweise enthält die Basenpaste zusätzlich 0,0001 bis 1 Gew.-%, bevorzugt 0,0005 bis 0,500 Gew.-% und besonders bevorzugt 0,0007 bis 0,020 Gew.-% einer oder mehrerer Übergangsmetallverbindungen. Angegeben ist die Gesamtmasse an Übergangsmetallverbindungen bezogen auf die Masse der Basenpaste.

Vorzugsweise weist die Basenpaste die folgende Zusammensetzung auf:
- 0,10 bis 5,00 Gew.-%, bevorzugt 0,50 bis 4,00 Gew.-%, besonders bevorzugt 1,00 bis 2,00 Gew.-% Thioharnstoffderivate,
- 0,0001 bis 1 Gew.-%, bevorzugt 0,0005 bis 0,5 Gew.-%, besonders bevorzugt 0,0007 bis 0,02 Gew.-% mindestens einer Übergangsmetallverbindung,
- 5 bis 95 Gew.-%, bevorzugt 10 bis 95 Gew.-% und besonders bevorzugt 10 bis 90 Gew.-% mindestens eines radikalisch polymerisierbaren Monomers,
- 0 bis 85 Gew.-% mindestens eines Füllstoffs, und
- ggf. 0,01 bis 5 Gew.-%, bevorzugt 0,1 bis 3 Gew.-% und besonders bevorzugt 0,1 bis 2 Gew.-% eines oder mehrere Additive.

Die obigen Angaben beziehen sich in allen Fällen auf die Masse der Basenpaste.

Die Katalysatorpaste enthält ein oder mehrere Hydroperoxide vorzugsweise in einer Gesamtmenge von 0,01 bis 5,0 Gew.-%, besonders bevorzugt 0,05 bis 4,0 Gew.-% und ganz besonders bevorzugt 0,1 bis 3,75 Gew.-%, bezogen auf die Masse der Katalysatorpaste.

Vorzugsweise weist die Katalysatorpaste die folgende Zusammensetzung auf:
- 0,01 bis 6 Gew.-%, bevorzugt 0,05 bis 5,0 Gew.-% und besonders bevorzugt 0,1 bis 4,0 Gew.-% mindestens eines Hydroperoxids, vorzugsweise CHP und/oder K220,
- 5 bis 95 Gew.-%, bevorzugt 10 bis 95 Gew.-% und besonders bevorzugt 10 bis 90 Gew.-% mindestens eines radikalisch polymerisierbaren Monomers,
- 0 bis 85 Gew.-% mindestens eines Füllstoffs, und
- ggf. 0,01 bis 5 Gew.-%, bevorzugt 0,1 bis 3 Gew.-% und besonders bevorzugt 0,1 bis 2 Gew.-% eines oder mehrere Additive, jeweils bezogen auf die Masse der Katalysatorpaste.

Die erfindungsgemäßen Werkstoffe haben nach dem Mischen von Katalysator- und Basenpaste vorzugsweise die folgende Gesamtzusammensetzung:
(a) 0,005 bis 3 Gew.-%, bevorzugt 0,025 bis 2,5 Gew.-% und besonders bevorzugt 0,05 bis 2,0 Gew.-% mindestens eines Hydroperoxids, vorzugsweise CHP und/oder K220,
(b) 0,005 bis 3,0 Gew.-%, bevorzugt 0,015 bis 2,125 Gew.-%, besonders bevorzugt 0,025 bis 1,5 Gew.-% Thioharnstoffderivate,
(c) 0,00005 bis 0,5 Gew.-%, bevorzugt 0,00025 bis 0,25 Gew.-%, besonders bevorzugt 0,00035 bis 0,01 Gew.-% mindestens einer Übergangsmetallverbindung,
(d) 5 bis 95 Gew.-%, bevorzugt 10 bis 95 Gew.-% und besonders bevorzugt 10 bis 90 Gew.-% mindestens eines radikalisch polymerisierbaren Monomers,
(e) 0 bis 85 Gew.-% mindestens eines Füllstoffs, und
(f) ggf. 0,01 bis 5 Gew.-%, bevorzugt 0,1 bis 3 Gew.-% und besonders bevorzugt 0,1 bis 2 Gew.-% eine oder mehrerer Additive, jeweils bezogen auf die Gesamtmasse der Zusammensetzung.

Der Füllstoffgehalt richtet sich nach dem gewünschten Anwendungszweck des Werkstoffs. Füllungskomposite haben vorzugsweise einen Füllstoffgehalt von 50 bis 85 Gew.-%, besonders bevorzugt 70 bis 80 Gew.-%. Materialien zur Herstellung provisorischer Kronen und Brücken weisen vorzugsweise einen Füllstoffgehalt von 40 bis 70 Gew.-%, besonders bevorzugt 45 bis 60 Gew.-%, und dentale Zemente von 10 bis 70 Gew.-%, besonders bevorzugt 60 bis 70 Gew.-% auf. Die Angaben beziehen sich auf die Gesamtmasse des Werkstoffs.

Besonders bevorzugt sind solche Zusammensetzungen, die aus den genannten Stoffen bestehen. Weiterhin sind solche Zusammensetzungen bevorzugt, bei denen die einzelnen Komponenten jeweils aus den oben genannten bevorzugten und besonders bevorzugten Stoffen ausgewählt sind. In allen Fällen kann jeweils eine einzelne Komponente oder eine Mischung von mehreren Komponenten eingesetzt werden, also beispielsweise eine Mischung von Monomeren.

Die erfindungsgemäßen Zusammensetzungen eignen sich besonders als Dentalwerkstoffe, insbesondere als dentale Zemente, Füllungskomposite und Verblendmaterialien sowie als Materialien zur Herstellung von Prothesen, künstlichen Zähnen, Inlays, Onlays, Kronen und Brücken, ganz besonders zur Herstellung provisorischer Kronen und Brücken. Die Zusammensetzungen eignen sich zur intraoralen Anwendung durch den Zahnarzt zur Restauration geschädigter Zähne, d.h. zur therapeutischen Anwendung, z.B. als dentale Zemente, Füllungskomposite und Verblendmaterialien. Sie können aber auch nicht-therapeutisch (extraoral) eingesetzt werden, beispielsweise bei der Herstellung oder Reparatur von Dentalrestaurationen, wie Prothesen, künstlichen Zähnen, Inlays, Onlays, Kronen und Brücken.

Die erfindungsgemäßen Zusammensetzungen eignen sich außerdem zur Herstellung von Formkörpern für dentale aber auch für nicht dentale Zwecke, die z.B. mittels Gießen, Pressen und insbesondere durch generative Verfahren wie den 3D Druck hergestellt werden können.

Zur Herstellung von provisorischen Kronen und Brücken wird zunächst ein Abdruck des oder der zu behandelnden Zähne angefertigt. Anschließend werden der oder die Zähne beschliffen und dann der Abdruck mit einem selbsthärtenden Restaurationsmaterial gefüllt. Das Applizieren des angemischten Materials erfolgt unter leichtem Druck aus einer Mischkanüle direkt in den Abdruck bzw. die Tiefziehfolie. Beim Einfüllen sollte die Kanüle in das Material eintauchen, um ein blasenfreies Befüllen des Abdrucks zu erreichen. Die präparierten Zähne können bei Bedarf zur blasenfreien Abbildung des Präparationsrandes mit dem Restaurationsmaterial umspritzt werden.

Der mit dem Material befüllte Abdruck wird anschließend auf die präparierten Zähne gedrückt. Nach etwa 1 bis 2 Minuten hat das Material einen hart-elastisch angehärteten Zustand und kann zusammen mit dem Abdruck aus dem Mund entnommen werden.

Nach dem Entfernen des Kunststoffprovisoriums aus der Situationsabformung oder ggf. vom Stumpf werden Überschüsse nach weiterer Aushärtung (ca. 4 bis 5 Minuten) mit rotierenden Instrumenten, z.B. mit einem kreuzverzahnten Hartmetallfräser, oder mit einem Skalpell entfernt. Die Inhibierungsschicht wird vorzugsweise mit Alkohol oder durch Polieren entfernt. Danach wird die Restauration vollständig aushärten gelassen.

Die Erfindung wird im Folgenden anhand von Ausführungsbeispielen und Figuren näher erläutert.
Figur 1 zeigt die Lagerstabilität von Pasten mit zwei unterschiedlichen Thioharnstoffderivaten. Die Verarbeitungszeit der Pasten nimmt mit der Lagerdauer zu, was ein Hinweis auf Instabilität ist. Die Pasten sind bei 37°C für ca. drei Monate stabil.
Figur 2 zeigt die Lagerstabilität von Pasten mit drei unterschiedlichen Thioharnstoffderivaten. Die Pasten sind selbst bei 50°C für 5 Monate stabil. Erst danach ist ein Abfall der Verarbeitungszeit zu erkennen.

### Ausführungsbeispiele

In den Ausführungsbeispielen werden die folgenden Materialien verwendet:
- DCP: Bis(methacryloyloxymethyl)tricyclo-[5.2.1.02,6]-decan (CAS-Nr. 43048-08-4)
- RM3: UDMA (CAS-Nr. 72869-86-4)
- SR348C: 2-[4-(2-Methacryloyloxyethoxyethoxy)-phenyl]-2-[4-(2-methacryloyl-oxyethoxy)-phenyl]-propan) (SR-348c, Firma Sartomer; enthält 3 Ethoxygruppen; CAS-Nr. 41637-38-1)
- CHP: Cumolhydroperoxid (CAS-Nr. 80-15-9)
- K220: 4-(2-Hydroperoxypropan-2-yl)phenyl propionat (CAS-Nr. 2515246-70-3)
- 2-MBI: 2-Mercaptobenzimidazol (CAS-Nr. 583-39-1)
- 1-Methyl-MBI: 1-Methyl-1H-benzimidazol-2-thiol (CAS-Nr. 2360-22-7)
- THPT: 3,4,5,6-Tetrahydro-2-pyrimidinthiol (CAS-Nr. 2055-46-1)
- K107: Hexanoylthioharnstoff (CAS-Nr. 41510-13-8)
- CuCI: Kupfer(I)chlorid (CAS-Nr. 7758-89-6)
- HDK2000: pyrogene Kieselsäure behandelt mit Trimethylsilan, BET-Oberfläche ca. 200 m²/g (Wacker Chemie AG)
- SG-SO100NCMP8: SiO₂ beschichtet mit Methacryloxypropyltrimethoxysilan (CAS-Nr. 7631-86-9)
- BHT: Butylhydroxytoluol (CAS-Nr. 128-37-0)
- Optamint^{®}: Geschmacksstoff (Fa. Symrise AG)

### Beispiel 1

### Bestimmung des Einflusses der Anzahl von Thioharnstoffderivaten auf die Lagerstabilität

Die in Tabelle 1 aufgeführten Werkstoffe wurden durch homogenes Mischen der in der Tabelle genannten Komponenten hergestellt. Es wurde jeweils eine Basenpaste (Base) und eine Katalysatorpaste (Kat) hergestellt. Zur Herstellung der Pasten wurden die jeweiligen Initiatorbestandteile und Additive in das Monomer gegeben und die Mischung anschließend für mehrere Stunden bis zur vollständigen Lösung der Feststoffe gerührt. Danach wurden die Füllstoffe zugegeben und in einem Zentrifugalmischer (SpeedMixer^{®}, Fa. Hauschild) homogen vermischt. Nach dem Vakuumieren der Pasten wurde diese getrennt in eine Doppelschubspritze gefüllt. Danach wurden Basen- und Katalysatorpaste jeweils im Volumenverhältnis 1:1 miteinander vermischt und die Verarbeitungszeit (VZ) nach der Norm EN ISO-4049 gemessen. Zur Bestimmung der mechanischen Eigenschaften der Werkstoffe wurden Prüfkörper hergestellt und deren Biegefestigkeit und Biege-E-Modul nach der Norm EN ISO-4049 (Dentistry - Polymer-based filling, restorative and luting materials) ermittelt. Zur Bestimmung der Dauer der elastischen Phase wurden Katalysator- und Basenpaste in einem Volumenverhältnis 1:1 vermischt. Nach dem Einsetzen der Härtung (Feststellung durch Druck auf dem Prüfkörper) wurde die Stoppuhr gestartet und wenn der Prüfkörper nicht mehr mit einem Skalpell geschnitten werden konnte gestoppt. Die Ergebnisse sind in Tabelle 3 angegeben.

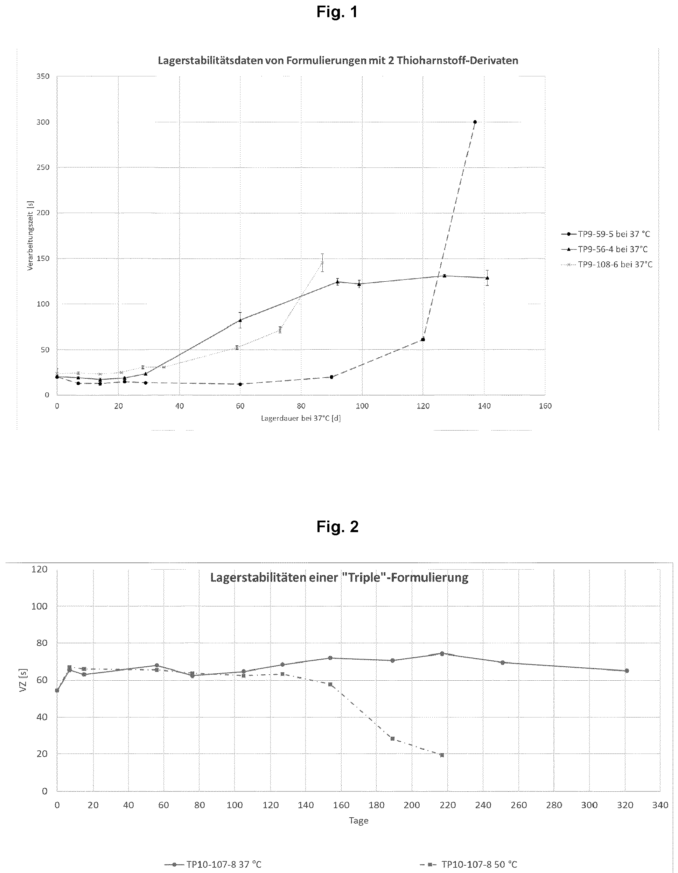

Zur Bestimmung der Lagerstabilität wurden die Pasten für fünf Monate bei 37°C bzw. 50°C gelagert. Die Verarbeitungszeit wurde periodisch gemessen. Die Ergebnisse sind in den Figuren 1 und 2 wiedergegeben. Figur 1 zeigt die Lagerstabilität von Pasten mit zwei und Fig. 2 die Lagerstabilität von Pasten mit drei unterschiedlichen Thioharnstoffderivaten. Fig. 1 zeigt, dass sich die Verarbeitungszeit der Pasten mit der Lagerdauer erhöht, was ein Hinweis auf Instabilität ist. Die Pasten mit zwei unterschiedlichen Thioharnstoffderivaten waren bei 37°C für ca. drei Monate stabil. Fig. 2 zeigt, dass Pasten mit drei unterschiedlichen Thioharnstoffderivaten selbst bei 50°C für 5 Monate stabil waren.

**Tabelle 1: Zusammensetzung der Katalysator- und Basenpasten (Angaben in Gew.-%)**

| **Komponente** | | **TP9-59-5*)** | | **TP9-56-4*)** | | **TP9-108-6*)** | | **TP10-107-8** | |
|---|---|---|---|---|---|---|---|---|---|
| | | Base | Kat | Base | Kat | Base | Kat | Base | Kat |
| **Monomer** | SR348C | 47.07 | 46.02 | 47.04 | 46.47 | 18.98 | 18.53 | 23.43 | 22.83 |
| | RM3 | | | | | 2372 | 23.23 | 17.92 | 17.87 |
| | DCP | | | | | 4.75 | 4.65 | 5.12 | 484 |
| **Peroxid** | CHP | | 2.51 | | 2.49 | | 2.52 | | 2.41 |
| **Thioharnstoff** | 2-MBI | 0.52 | | 0.50 | | 0.49 | | | |
| | K107 | 0.49 | | 0.49 | | 0.49 | | 0.48 | |
| | THPT | | | | | | | 0.24 | |
| | 2-Imidazolidinthion | | | | | | | 0.14 | |
| **Cu-Salz** | CuCI | 0.02 | | 0.02 | | 0.02 | | 0.01 | |
| **Aroma** | Optamint^{®} | 0.49 | | 0.96 | | 0.50 | | 0.49 | |
| **Stabilisator** | BHT | 0.05 | 0.05 | 0.05 | 0.05 | 0.07 | 0.06 | 0.05 | 0.05 |
| **Füllstoff** | HDK 2000 | 296 | 300 | 3.00 | 3.01 | 300 | 3.01 | 5.02 | 4.99 |
| | SG-SO1 OONCMP8 | 48.41 | 48.41 | 47.95 | 4799 | 47.98 | 4799 | 47.10 | 47.01 |
| **Summe** | | 10000 | 10000 | 10000 | 10000 | 10000 | 10000 | 10000 | 100.00 |

| | | | | | | | | | |
|---|---|---|---|---|---|---|---|---|---|
| *) Vergleichsbeispiel | | | | | | | | | |

### Beispiel 2

### Bestimmung der mechanischen Eigenschaften von Restaurationsmaterialien

Analog zu Beispiel 1 wurden die in Tabelle 2 beschriebenen Werkstoffe hergestellt. Anschließend wurden die Verarbeitungszeit, die Dauer der elastischen Phase und die mechanischen Eigenschaften auf die in Beispiel 1 beschriebene Weise bestimmt. Die Ergebnisse sind in Tabelle 3 angegeben. Die Werkstoffe Nr. 1 und Nr. 2 enthalten jeweils nur zwei Thioharnstoffderivate. Diese Werkstoffe weisen eine gute Biegefestigkeit, ein gutes Biegemodul und eine ausreichend lange elastische Phase auf. Allerdings ist die Verarbeitungszeit relativ kurz und die Lagerstabilität unbefriedigend (Figur 1). Die übrigen Werkstoffe enthalten jeweils drei unterschiedliche Thioharnstoffderivate. Diese Werkstoffe zeichnen sich bei vergleichbaren mechanischen Eigenschaften und vergleichbarer Dauer der elastischen Phase durch eine verlängerte Verarbeitungszeit aus.

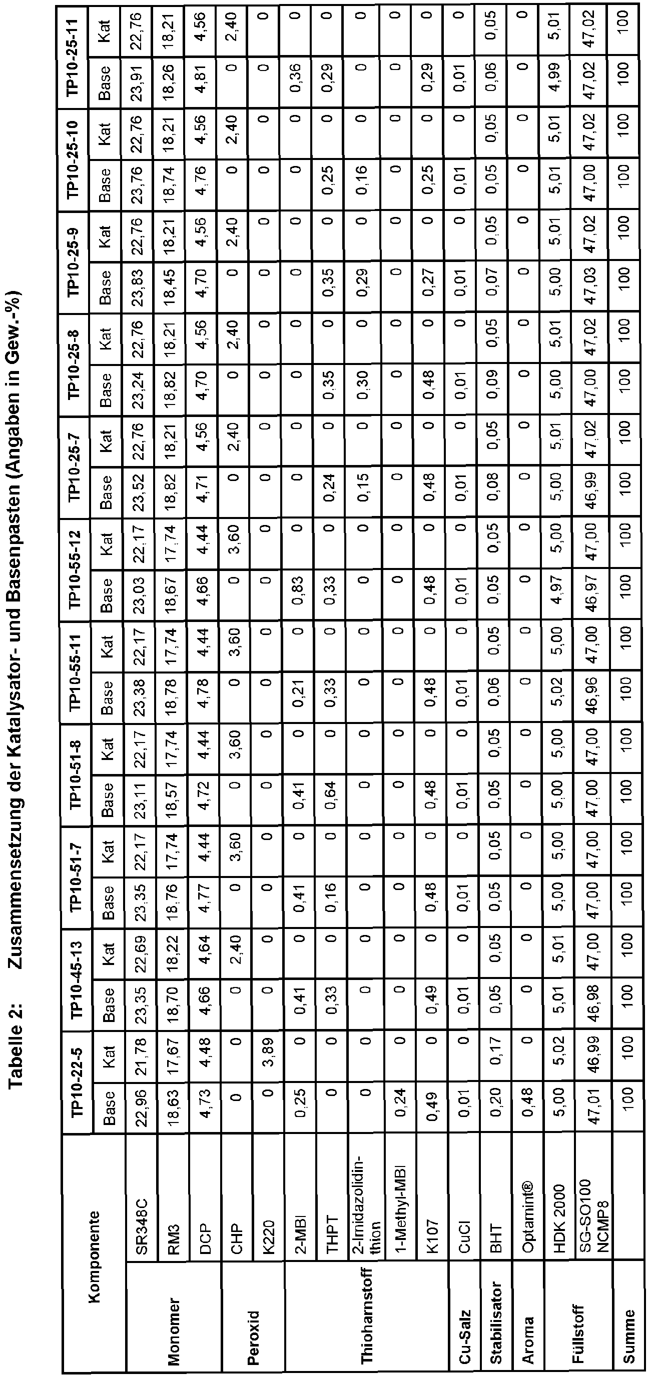

**Tabelle 3: Eigenschaften der Werkstoffe**

| **Nr.** | **Werkstoff** | **VZ[s]** | **Biegefestigkeit [MPa]** | **Biege-Modul [MPa]** | **Dauer der elastischen Phase [s]** |
|---|---|---|---|---|---|
| **1** | TP9-59-5*) | 20 | 90 | 3858 | 80 |
| **2** | TP9-108-6*) | 24 | 95 | 4114 | 87 |
| **3** | TP10-22-5 | 35 | 76 | 2709 | 100 |
| **4** | TP10-25-7 | 47 | 128 | 4800 | 100 |
| **5** | TP10-25-8 | 52 | 117 | 4376 | 100 |
| **6** | TP10-25-9 | 56 | 110 | 4436 | 70 |
| **7** | TP10-25-10 | 52 | 97 | 4221 | 90 |
| **8** | TP10-25-11 | 56 | 83 | 3251 | 80 |
| **9** | TP10-45-13 | 71 | 83 | 3107 | 90 |
| **10** | TP10-51-7 | 66 | 91 | 4249 | 100 |
| **11** | TP10-51-8 | 53 | 90 | 4414 | 80 |
| **12** | TP10-55-11 | 53 | 97 | 4490 | 60 |
| **13** | TP10-55-12 | 42 | 95 | 4544 | 70 |

| | | | | | |
|---|---|---|---|---|---|
| *) Vergleichsbeispiel aus Beispiel 1 | | | | | |

## Patentansprüche

1. Radikalisch polymerisierbarer Dentalwerkstoff, der als Initiatorsystem für die radikalische Polymerisation eine Kombination aus einem Thioharnstoffderivat und einem Hydroperoxid enthält, **dadurch gekennzeichnet, dass** er mehrere cyclische und ein oder mehrere acyclische Thioharnstoffderivate enthält.

2. Dentalwerkstoff nach Anspruch 1, der mindestens ein cyclisches Thioharnstoffderivat der Formel (I) enthält: 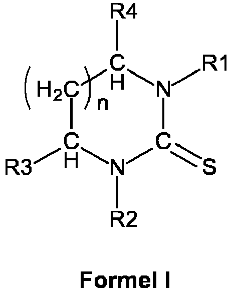 in der:
R¹, R² jeweils H oder ein C₁-C₄₋Alkylrest sind, wobei mindestens einer dieser Reste H ist;
R³, R⁴ unabhängig voneinander jeweils H, ein C₁-C₄₋Alkylrest oder ein C₁-C₄Alkoxyrest sind oder zusammen mit den Kohlenstoffatomen, an die sie gebunden sind, und dem dazwischen liegenden Kohlenstoffatom einen sechsgliedrigen, carbocyclischen, aliphatischen oder aromatischen Ring bilden, der durch einen oder mehrere, vorzugsweise 1 oder 2, C₁-C₄-Alkylreste und/oder C₁-C₄-Alkoxyreste substituiert sein kann;
n 0, 1, 2 oder 3, vorzugsweise 0 oder 1 ist.

3. Dentalwerkstoff nach Anspruch 1 oder 2, der mindestens ein acylisches Thioharnstoffderivat der Formel (II) 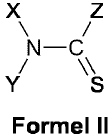 in der
X H oder Y ist,
Y ein Alkylrest mit 1 bis 8 Kohlenstoffatomen, ein Cycloalkylrest mit 5 oder 6 Kohlenstoffatomen, ein Chlor-, Hydroxy- oder Mercapto-substituierter Alkylrest mit 1 bis 8 Kohlenstoffatomen, ein Alkenylrest mit 3 bis 4 Kohlenstoffatomen, ein Arylrest mit 6 bis 8 Kohlenstoffatomen, ein Chlor-, Hydroxy-, Methoxy- oder Sulfonyl-substituierter Phenylrest, ein Acylrest mit 2 bis 8 Kohlenstoff-atomen, ein Chlor- oder Methoxy-substituierter Acylrest, ein Aralkylrest mit 7 bis 8 Kohlenstoffatomen oder ein Chlor- oder Methoxy-substituierter Aralkylrest ist, und
Z NH₂, NHX oder NX₂ ist, und/oder
der Formel (III) enthält, 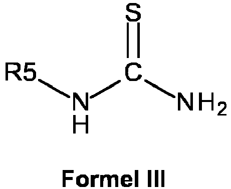 in der
R⁵ ein C₁-C₁₂-Alkylrest, vorzugsweise ein C₂-C₁₀-Alkylrest, besonders bevorzugt ein C₃-C₈-Alkylrest und ganz besonders bevorzugt ein C₄-C₆-Alkylrest, ein C₁-C₁₂-Alkenrest, vorzugsweise ein C₂-C₁₀-Alkenrest, besonders bevorzugt ein C₃-C₈-Alkenrest und ganz besonders bevorzugt ein C₄-C₆-Alkenrest, ein C₁-C₁₂-Acylrest, vorzugsweise ein C₂-C₁₀-Acylrest, besonders bevorzugt ein C₃-C₈-Acylrest und ganz besonders bevorzugt ein C₄-C₆-Acylrest, ein Pyridyl- oder Phenylrest ist.

4. Dentalwerkstoff nach einem der vorhergehenden Ansprüche, der als cyclisches Thioharnstoffderivat 3,4,5,6-Tetrahydro-2-pyrimidinthiol, 2-Imidazolidinthion, 2-Mercaptobenzimidazol, 1-Methyl-1H-benzimidazol-2-thiol und/oder 2-Mercapto-5-methoxybenzimidazol, und als acyclisches Thioharnstoffderivat Hexanoylthioharnstoff enthält.

5. Dentalwerkstoff nach einem der Ansprüche 1 bis 4, der als Hydroperoxid eine Verbindung der Formel R⁶-(OOH)ₘ, in der R⁶ ein aliphatischer oder aromatischer Kohlenwasserstoffrest und m 1 oder 2 ist, und/oder
eine Verbindung der Formel (IV) enthält, 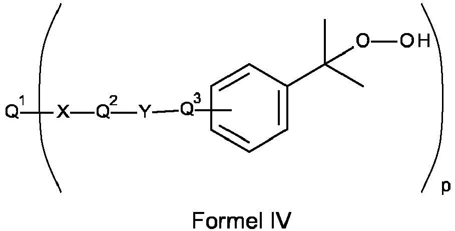 in der die Variablen die folgenden Bedeutungen haben:
Q¹ ein p-wertiger, aromatischer, aliphatischer, linearer oder verzweigter C₁-C₁₄-Kohlenwasserstoffrest, der durch ein oder mehrere S- und/oder O-Atome unterbrochen sein kann und der unsubstituiert oder durch einen oder mehrere Substituenten, die vorzugsweise aus -OH, -OR⁷, -Cl und -Br ausgewählt sind, substituiert sein kann, wobei R⁷ein aliphatischer, linearer oder verzweigter C₁-C₁₀-Kohlenwassrstoffrest ist,
X, Y unabhängig voneinander jeweils entfällt, -O-, -COO-; -CONR⁸-, oder -O-CO-NR⁹-, wobei R⁸ und R⁹ unabhängig voneinander für H oder einen C₁-C₅-Alkylrest, vorzugsweise für H, Methyl und/oder Ethyl, besonders bevorzugt H stehen, und wobei X und Y vorzugsweise nicht gleichzeitig entfallen,
Q² entfällt, ein aliphatischer, linearer oder verzweigter C₁-C₁₄-Alkylen-Rest, der durch S- und/oder O-Atome unterbrochen sein kann und der unsubstituiert oder durch -OH, -OR¹⁰, -CI und/oder -Br substituiert sein kann, wobei R¹⁰ ein aliphatischer, linearer oder verzweigter C₁₋C₁₀-Kohlenwasserstoffrest ist,
Q³ eine C₁-C₃Alkylengruppe oder entfällt, vorzugsweise -CH₂- oder entfällt, wobei X und/oder Y entfällt, wenn Q² entfällt,
p 1, 2, 3 oder 4, und wobei die Substitution am Aromaten in 2-, 3- oder 4-Stellung erfolgt, relativ zur Cumolhydroperoxidgruppe.

6. Dentalwerkstoff nach Anspruch 5, der als Hydroperoxid t-Amylhydroperoxid, 1,1,3,3-Tetramethylbutylhydroperoxid, t-Butylhydroperoxid, t-Hexylhydroperoxid, 2,5-Dimethyl-2,5-di(hydroperoxy)hexan, Diisopropylbenzolmonohydroperoxid, Paramenthanhydroperoxid, p-Isopropylcumolhydroperoxid, 4-(2-Hydroperoxypropan-2-yl)phenylpropionat oder eine Mischung davon, vorzugsweise Cumolhydroperoxid (CHP) und/oder 4-(2-Hydroperoxypropan-2-yl)phenylpropionat enthält.

7. Dentalwerkstoff nach einem der vorangehenden Ansprüche, der zusätzlich eine Übergangsmetallverbindung enthält, vorzugsweise eine Verbindung des Eisens, Kobalts, Nickels, Mangans oder eine Mischung davon, besonders bevorzugt des Kupfers und ganz besonders bevorzugt von Cu(I).

8. Dentalwerkstoff nach einem der vorangehenden Ansprüche, der zwei Komponenten umfasst, wobei die erste Komponente 0,01 bis 5 Gew.-%, bevorzugt 0,05 bis 4,0 Gew.-% und besonders bevorzugt 0,1 bis 3,75 Gew.-% Hydroperoxid, bezogen auf die Masse der ersten Komponente enthält, und wobei die zweite Komponente 50 bis 400 mol-%, bevorzugt 75 bis 300 mol-% und besonders bevorzugt 100 bis 200 mol.% Thioharnstoffderivat, bezogen auf die molare Menge an Hydroperoxid in der ersten Komponente enthält.

9. Dentalwerkstoff nach einem der vorangehenden Ansprüche, der zusätzlich mindestens ein radikalisch polymerisierbares Monomer enthält, vorzugsweise mindestens ein mono- oder multifunktionelles (Meth)acrylat, besonders bevorzugt mindestens ein Dimethacrylat oder eine Mischung von Mono- und Dimethacrylaten.

10. Dentalwerkstoff nach Anspruch 9, der als radikalisch polymerisierbares Monomer Methyl-, Ethyl-, 2-Hydroxyethyl-, Butyl-, Benzyl-, Tetrahydrofurfuryl- oder Isobornyl(meth)acrylat, p-Cumyl-phenoxyethylenglycolmethacrylat (CMP-1E), 2-(2-Biphenyloxy)-ethylmethacrylat, Bisphenol-A-dimethacrylat, Bis-GMA (ein Additionsprodukt aus Methacrylsäure und Bisphenol-A-diglycidylether), ethoxy- oder propoxyliertes Bisphenol-A-Dimethacrylat, 2-[4-(2-Methacryloyloxyethoxyethoxy)phenyl]-2-[4-(2-methacryloyloxyethoxy)phenyl]propan) (SR-348c), 2,2-Bis[4-(2-methacryloxy-propoxy)phenyl]propan, UDMA (ein Additionsprodukt aus 2-Hydroxyethylmethacrylat und 2,2,4-Trimethylhexamethylen-1,6-diisocyanat), V-380 (ein Additionsprodukt aus einer Mischung von 0,7 mol 2-Hydroxyethylmethacrylat und 0,3 mol 2-Hydroxypropylmethacrylat mit 1 mol α,α,α',α'-Tetramethyl-m-xylylen-diisocyanat), Di-, Tri- oder Tetraethylenglycoldimethacrylat, Trimethylolpropantrimethacrylat, Pentaerythrittetramethacrylat, Glycerindi- und -trimethacrylat, 1,4-Butandioldimethacrylat, 1,10-Decandioldimethacrylat (D₃MA), Bis(methacryloyloxymethyl)tricyclo-[5.2.1.02,6]decan (DCP), ein Polyethylenglycol- oder Polypropylenglycoldimethacrylat, Polyethylenglycol-200- ,Polyethylenglycol-400-dimethacrylat (PEG-200- oder PEG-400-DMA), 1,12-Dodecandioldimethacrylat oder eine Mischung davon enthält.

11. Dentalwerkstoff nach einem der vorangehenden Ansprüche, der zusätzlich mindestens einen organischen oder anorganischen Füllstoff enthält, vorzugsweise ein Oxid, wie SiO₂, ZrO₂ und TiO₂ oder ein Mischoxid aus SiO₂, ZrO₂, ZnO und/oder TiO₂, einen nanopartikulären oder mikrofeinen Füllstoff, wie pyrogene Kieselsäure oder Fällungskieselsäure, Glaspulver, wie Quarz-, Glaskeramik- oder röntgenopakes Glaspulver, vorzugsweise Barium- oder Strontiumaluminiumsilikatglaspulver, einen röntgenopaken Füllstoff, wie Ytterbiumtrifluorid, Tantal(V)-oxid, Bariumsulfat, ein Mischoxid von SiO₂ mit Ytterbium(III)-oxid oder Tantal(V)-oxid, ein gemahlenes Präpolymerisat oder eine Perlpolymerisat.

12. Dentalwerkstoff nach einem der vorangehenden Ansprüche, der
(a) 0,005 bis 3 Gew.-%, bevorzugt 0,025 bis 2,5 Gew.-% und besonders bevorzugt 0,05 bis 2,0 Gew.-% mindestens eines Hydroperoxids, vorzugsweise Cumolhydroperoxid und/oder 4-(2-hydroperoxypropan-2-yl)phenyl propionat,
(b) 0,005 bis 3,0 Gew.-%, bevorzugt 0,015 bis 2,125 Gew.-%, besonders bevorzugt 0,025 bis 1,5 Gew.-% Thioharnstoffderivate,
(c) 0,00005 bis 0,5 Gew.-%, bevorzugt 0,00025 bis 0,25 Gew.-%, besonders bevorzugt 0,00035 bis 0,01 Gew.-% mindestens einer Übergangsmetallverbindung,
(d) 5 bis 95 Gew.-%, bevorzugt 10 bis 95 Gew.-% und besonders bevorzugt 10 bis 90 Gew.-% mindestens eines radikalisch polymerisierbaren Monomers,
(e) 0 bis 85 Gew.-% mindestens eines Füllstoffs, und
(f) ggf. 0,01 bis 5 Gew.-%, bevorzugt 0,1 bis 3 Gew.-% und besonders bevorzugt 0,1 bis 2 Gew.-% eine oder mehrerer Additive enthält, jeweils bezogen auf die Gesamtmasse der Zusammensetzung.

13. Dentalwerkstoff nach einem der vorangehenden Ansprüche zur therapeutischen Anwendung.

14. Verwendung eines Dentalwerkstoffs gemäß einem der Ansprüche 1 bis 12 zur extraoralen Herstellung oder Reparatur von Dentalrestaurationen, wie Prothesen, künstlichen Zähnen, Inlays, Onlays, Kronen, Brücken und Vollprothesen.

## Claims

1. Radically polymerizable dental material, which comprises a combination of a thiourea derivative and a hydroperoxide as initiator system for the radical polymerization, **characterized in that** it comprises several cyclic and one or more acyclic thiourea derivatives.

2. Dental material according to claim 1, which comprises at least one cyclic thiourea derivative of Formula (I): 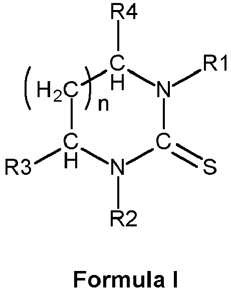 in which:
R¹, R² in each case are H or a C₁-C₄ alkyl radical, wherein at least one of these radicals is H;
R³, R⁴ independently of each other in each case are H, a C₁-C₄ alkyl radical or a C₁-C₄ alkoxy radical or, together with the carbon atoms to which they are bonded and the carbon atom lying in between, form a six-membered, carbocyclic, aliphatic or aromatic ring, which can be substituted by one or more, preferably 1 or 2, C₁-C₄ alkyl radicals and/or C₁-C₄ alkoxy radicals;
n is 0, 1, 2 or 3, preferably 0 or 1.

3. Dental material according to claim 1 or 2, which comprises at least one acyclic thiourea derivative of Formula (II): 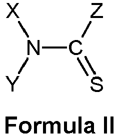 in which
X is H or Y,
Y is an alkyl radical with 1 to 8 carbon atoms, a cycloalkyl radical with 5 or 6 carbon atoms, a chlorine-, hydroxy- or mercapto-substituted alkyl radical with 1 to 8 carbon atoms, an alkenyl radical with 3 to 4 carbon atoms, an aryl radical with 6 to 8 carbon atoms, a chlorine-, hydroxy-, methoxy- or sulfonyl-substituted phenyl radical, an acyl radical with 2 to 8 carbon atoms, a chlorine-or methoxy-substituted acyl radical, an aralkyl radical with 7 to 8 carbon atoms or a chlorine- or methoxy-substituted aralkyl radical, and
Z is NH₂, NHX or NX₂, and/or
of Formula (III): 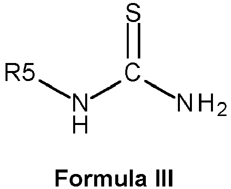 in which
R⁵ is a C₁-C₁₂ alkyl radical, preferably a C₂-C₁₀ alkyl radical, particularly preferably a C₃-C₈ alkyl radical and quite particularly preferably a C₄-C₆ alkyl radical, a C₁-C₁₂ alkene radical, preferably a C₂-C₁₀ alkene radical, particularly preferably a C₃-C₈ alkene radical and quite particularly preferably a C₄-C₆ alkene radical, a C₁-C₁₂ acyl radical, preferably a C₂-C₁₀ acyl radical, particularly preferably a C₃-C₈ acyl radical and quite particularly preferably a C₄-C₆ acyl radical, a pyridyl or phenyl radical.

4. Dental material according to any one of the preceding claims, which comprises 3,4,5,6-tetrahydro-2-pyrimidinethiol, 2-imidazolidinethione, 2-mercaptobenzimidazole, 1-methyl-1H-benzimidazole-2-thiol and/or 2-mercapto-5-methoxybenzimidazole as cyclic thiourea derivative, and hexanoyl thiourea as acyclic thiourea derivative.

5. Dental material according to any one of claims 1 to 4, which comprises a compound of the formula R⁶-(OOH)ₘ as hydroperoxide, in which R⁶ is an aliphatic or aromatic hydrocarbon radical and m is 1 or 2, and/or
a compound of Formula (IV), 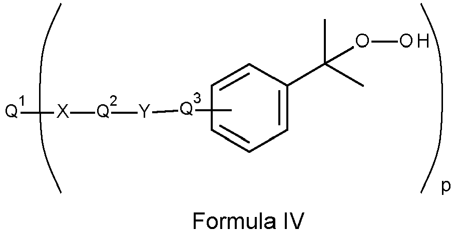 in which the variables have the following meanings:
Q¹ an p-valent, aromatic, aliphatic, linear or branched C₁-C₁₄ hydrocarbon radical, which can be interrupted by one or more S and/or O atoms and which can be unsubstituted or substituted by one or more substituents which are preferably selected from -OH, -OR⁷, -CI and -Br, wherein R⁷ is an aliphatic, linear or branched C₁-C₁₀ hydrocarbon radical,
X, Y independently of each other is in each case absent, -O-, -COO-; -CONR⁸-, or -O-CO-NR⁹-, wherein R⁸ and R⁹ independently of each other represent H or a C₁-C₅ alkyl radical, preferably H, methyl and/or ethyl, particularly preferably H, and wherein X and Y are preferably not absent at the same time,
Q² is absent, an aliphatic, linear or branched C₁-C₁₄ alkylene radical, which can be interrupted by S and/or O atoms and which can be unsubstituted or substituted by -OH, -OR¹⁰, -Cl and/or -Br, wherein R¹⁰ is an aliphatic, linear or branched C₁-C₁₀ hydrocarbon radical,
Q³ a C₁-C₃ alkylene group or is absent, preferably -CH₂- or is absent, wherein X and/or Y is absent if Q² is absent,
p 1, 2, 3 or 4, and wherein the substitution on the aromatic compound takes place in position 2, 3 or 4, relative to the cumene hydroperoxide group.

6. Dental material according to claim 5, which comprises t-amyl hydroperoxide, 1,1,3,3-tetramethylbutyl hydroperoxide, t-butyl hydroperoxide, t-hexyl hydroperoxide, 2,5-dimethyl-2,5-di(hydroperoxy)hexane, diisopropylbenzene monohydroperoxide, para-menthane hydroperoxide, p-isopropylcumene hydroperoxide, 4-(2-hydroperoxy-propan-2-yl)phenyl propionate or a mixture thereof, preferably cumene hydroperoxide (CHP) and/or 4-(2-hydroperoxypropan-2-yl)phenyl propionate, as hydroperoxide.

7. Dental material according to any one of the preceding claims, which additionally comprises a transition metal compound, preferably a compound of iron, cobalt, nickel, manganese or a mixture thereof, particularly preferably of copper and quite particularly preferably of Cu(I).

8. Dental material according to any one of the preceding claims, which comprises two components, wherein the first component comprises 0.01 to 5 wt.-%, preferably 0.05 to 4.0 wt.-% and particularly preferably 0.1 to 3.75 wt.-% hydroperoxide, relative to the mass of the first component, and wherein the second component comprises 50 to 400 mol-%, preferably 75 to 300 mol-% and particularly preferably 100 to 200 mol.-% thiourea derivative, relative to the molar quantity of hydroperoxide in the first component.

9. Dental material according to any one of the preceding claims, which additionally comprises at least one radically polymerizable monomer, preferably at least one mono- or multifunctional (meth)acrylate, particularly preferably at least one dimethacrylate or a mixture of mono- and dimethacrylates.

10. Dental material according to claim 9, which comprises methyl, ethyl, 2-hydroxyethyl, butyl, benzyl, tetrahydrofurfuryl or isobornyl (meth)acrylate, p-cumylphenoxyethylene glycol methacrylate (CMP-1E), 2-(2-biphenyloxy)ethyl methacrylate, bisphenol A dimethacrylate, bis-GMA (an addition product of methacrylic acid and bisphenol A diglycidyl ether), ethoxylated or propoxylated bisphenol A dimethacrylate, 2-[4-(2-methacryloyloxyethoxyethoxy)phenyl]-2-[4-(2-methacryloyloxyethoxy)phenyl]propane) (SR-348c), 2,2-bis[4-(2-methacryloxypropoxy)phenyl]propane, UDMA (an addition product of 2-hydroxyethyl methacrylate and 2,2,4-trimethylhexamethylene-1,6-diisocyanate), V-380 (an addition product of a mixture of 0.7 mol 2-hydroxyethyl methacrylate and 0.3 mol 2-hydroxypropyl methacrylate with 1 mol a,a,a',a'-tetramethyl-m-xylylene diisocyanate), di-, tri- or tetraethylene glycol dimethacrylate, trimethylolpropane trimethacrylate, pentaerythritol tetramethacrylate, glycerol di- and trimethacrylate, 1,4-butanediol dimethacrylate, 1,10-decanediol dimethacrylate (D₃MA), bis-(methacryloyloxymethyl)tricyclo-[5.2.1.02,6]decane (DCP), a polyethylene glycol or polypropylene glycol dimethacrylate, polyethylene glycol 200 dimethacrylate, polyethylene glycol 400 dimethacrylate (PEG 200 DMA or PEG 400 DMA), 1,12-dodecanediol dimethacrylate or a mixture thereof as radically polymerizable monomer.

11. Dental material according to any one of the preceding claims, which additionally comprises at least one organic or inorganic filler, preferably an oxide, such as SiO₂, ZrO₂ and TiO₂ or a mixed oxide of SiO₂, ZrO₂, ZnO and/or TiO₂, a nanoparticulate or microfine filler, such as pyrogenic silica or precipitated silica, glass powders, such as quartz, glass ceramic or radiopaque glass powder, preferably barium or strontium aluminium silicate glass powder, a radiopaque filler, such as ytterbium trifluoride, tantalum(V) oxide, barium sulfate, a mixed oxide of SiO₂ with ytterbium(III) oxide or tantalum(V) oxide, a ground prepolymer or a pearl polymer.

12. Dental material according to any one of the preceding claims, which comprises
(a) 0.005 to 3 wt.-%, preferably 0.025 to 2.5 wt.-% and particularly preferably 0.05 to 2.0 wt.-% of at least one hydroperoxide, preferably cumene hydroperoxide and/or 4-(2-hydroperoxypropan-2-yl)phenyl propionate,
(b) 0.005 to 3.0 wt.-%, preferably 0.015 to 2.125 wt.-%, particularly preferably 0.025 to 1.5 wt.-% thiourea derivatives,
(c) 0.00005 to 0.5 wt.-%, preferably 0.00025 to 0.25 wt.-%, particularly preferably 0.00035 to 0.01 wt.-% of at least one transition metal compound,
(d) 5 to 95 wt.-%, preferably 10 to 95 wt.-% and particularly preferably 10 to 90 wt.-% of at least one radically polymerizable monomer,
(e) 0 to 85 wt.-% at least one filler, and
(f) optionally 0.01 to 5 wt.-%, preferably 0.1 to 3 wt.-% and particularly preferably 0.1 to 2 wt.-% of one or more additives, in each case relative to the total mass of the composition.

13. Dental material according to any one of the preceding claims for therapeutic application.

14. Use of a dental material according to one of claims 1 to 12 for the extraoral production or repair of dental restorations, such as prostheses, artificial teeth, inlays, onlays, crowns, bridges and full dentures.

## Revendications

1. Matériau de dentisterie, polymérisable par voie radicalaire, qui contient, en tant que système d'amorçage de polymérisation par voie radicalaire, une association d'un dérivé de thiourée et d'un hydroperoxyde, **caractérisé en ce qu'**il contient plusieurs dérivés cycliques de thiourée et un ou plusieurs dérivé(s) acyclique(s) de thiourée

2. Matériau de dentisterie conforme à la revendication 1, qui contient au moins un dérivé cyclique de thiourée de formule (1) : 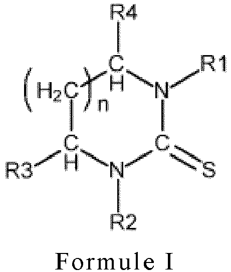 dans laquelle
- R¹ et R² représentent chacun un atome d'hydrogène ou un groupe alkyle en C₁-C₄, étant entendu qu'au moins l'un de ces symboles représente un atome d'hydrogène,
- R³ et R⁴ représentent chacun, indépendamment l'un de l'autre, un atome d'hydrogène, un groupe alkyle en C₁-C₄, ou un groupe alcoxy en C₁-C₄, ou bien des entités qui forment, conjointement avec les atomes de carbone auxquels elles sont liées et avec les atomes de carbone se trouvant entre ceux-ci, un cycle carbocyclique à six chaînons, aliphatique ou aromatique, lequel peut porter, en tant que substituant(s), un ou plusieurs, de préférence un ou deux, groupe(s) alkyle en C₁-C₄ et/ou alcoxy en C₁-C₄,
- et l'indice n vaut 0, 1, 2 ou 3, de préférence 0 ou 1.

3. Matériau de dentisterie conforme à la revendication 1 ou 2, qui contient au moins un dérivé acyclique de thiourée de formule (II) : 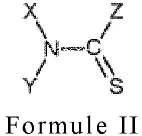 dans laquelle
- X représente un atome d'hydrogène ou un groupe symbolisé par Y,
- Y représente un groupe alkyle comportant 1 à 8 atomes de carbone, un groupe cycloalkyle comportant 5 ou 6 atomes de carbone, un groupe alkyle comportant 1 à 8 atomes de carbone et porteur de substituant(s) chloro, hydroxy ou mercapto, un groupe alcényle comportant 3 à 4 atomes de carbone, un groupe aryle comportant 6 à 8 atomes de carbone, un groupe phényle porteur de substituant(s) chloro, hydroxy, méthoxy ou sulfonyle, un groupe acyle comportant 2 à 8 atomes de carbone, un groupe acyle porteur de substituant(s) chloro ou méthoxy, un groupe aralkyle comportant 7 à 8 atomes de carbone, ou un groupe aralkyle porteur de substituant(s) chloro ou méthoxy,
- et Z représente un groupe symbolisé par NH₂, NHX ou NX₂,
et/ou de formule (III) : 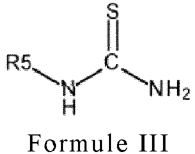 dans laquelle
- R⁵ représente un groupe alkyle en C₁-C₁₂, de préférence un groupe alkyle en C₂-C₁₀, mieux encore un groupe alkyle en C₃-C₈, et surtout un groupe alkyle en C₄-C₆,
un groupe alcényle en C₁-C₁₂, de préférence un groupe alcényle en C₂-C₁₀, mieux encore un groupe alcényle en C₃-C₈, et surtout un groupe alcényle en C₄-C₆,
un groupe acyle en C₁-C₁₂, de préférence un groupe acyle en C₂-C₁₀, mieux encore un groupe acyle en C₃-C₈, et surtout un groupe acyle en C₄-C₆,
ou un groupe pyridyle ou phényle.

4. Matériau de dentisterie conforme à l'une des revendications précédentes, qui contient, en tant que dérivé cyclique de thiourée, du 3,4,5,6-tétrahydro-pyrimidine-2-thiol, de l'imidazolidine-2-thione, du 2-mercapto-benzimidazole, du 1-méthyl-1H-benzimidazole-2-thiol et/ou du 2-mercapto-5-méthoxy-benzimidazole, ainsi que, en tant que dérivé acyclique de thiourée, de l'hexanoyl-thiourée.

5. Matériau de dentisterie conforme à l'une des revendications 1 à 4, qui contient, en tant qu'hydroperoxyde, un composé de formule R⁶-(OOH)ₘ dans laquelle R⁶ représente un groupe hydrocarboné aliphatique ou aromatique et l'indice m vaut 1 ou 2,
et/ou un composé de formule (IV) 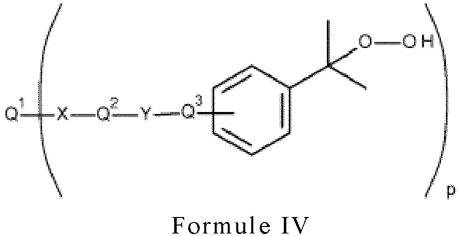 dans laquelle les symboles ont les significations suivantes :
- Q¹ représente un groupe hydrocarboné en C₁-C₁₄, de valence p, aromatique ou aliphatique linéaire ou ramifié, qui peut être interrompu par un ou plusieurs atome(s) de soufre et/ou d'oxygène et qui peut ne porter aucun substituant ou porter un ou plusieurs substituant(s) choisi(s) de préférence parmi ceux symbolisés par -OH, -OR⁷, Cl et -Br où R⁷ représente un groupe hydrocarboné aliphatique en C₁-C₁₀, linéaire ou ramifié,
- X et Y, indépendamment l'un de l'autre, ne représentent rien ou représentent chacun un fragment symbolisé par -O-, -CO-O-, -CO-NR⁸- ou -O-CO-NR⁹- où R⁸ et R⁹ représentent chacun, indépendamment l'un de l'autre, un atome d'hydrogène ou un groupe alkyle en C₁-C₅, et de préférence un atome d'hydrogène, un groupe méthyle et/ou un groupe éthyle, étant entendu que de préférence, X et Y ne représentent pas rien simultanément,
- Q² ne représente rien ou représente un groupe alcanediyle en C₁-C₁₄ aliphatique linéaire ou ramifié, qui peut être interrompu par un ou des atome(s) de soufre et/ou d'oxygène et qui peut ne porter aucun substituant ou porter un ou des substituant(s) symbolisés par -OH, -OR¹⁰, -Cl et/ou -Br, où R¹⁰ représente un groupe hydrocarboné aliphatique en C₁-C₁₀, linéaire ou ramifié,
- Q³ représente un groupe alcanediyle en C₁-C₃ ou ne représente rien, et de préférence représente un groupe -CH₂- ou ne représente rien,
- étant entendu que X et/ou Y ne représente(nt) rien si Q² ne représente rien,
- et l'indice p vaut 1, 2, 3 ou 4,
étant entendu que la substitution sur les cycles aromatiques, dans les groupes dérivés de l'hydroperoxy-cumène, intervient en position 2, 3 ou 4.

6. Matériau de dentisterie conforme à la revendication 5, qui contient, en tant qu'hydroperoxyde, de l'hydroperoxyde de tertioamyle, de l'hydroperoxyde de 1,1,3,3-tétraméthyl-butyle, de l'hydroperoxyde de tertiobutyle, de l'hydroperoxyde de tertiohexyle, du 2,5-diméthyl-2,5-dihydroperoxy-hexane, du monohydroperoxyde de diisopropyl-benzène, de l'hydroperoxyde de para-menthane, de l'hydroperoxyde de para-isopropyl-cumène, du propionate de 4-(2-hydroperoxy-prop-2-yl)-phényle, ou un mélange de ces composés, et de préférence, de l'hydroperoxyde de cumène (HPC) et/ou du propionate de 4-(2-hydroperoxy-prop-2-yl)-phényle.

7. Matériau de dentisterie conforme à l'une des revendications précédentes, qui contient en outre un composé d'un métal de transition, de préférence un composé du fer, du cobalt, du nickel, du manganèse ou un mélange de tels composés, mais en particulier un composé du cuivre et tout particulièrement de cuivre-1.

8. Matériau de dentisterie conforme à l'une des revendications précédentes, qui comprend deux composants, dont le premier composant contient de 0,01 à 5 % en poids, de préférence de 0,05 à 4,0 % en poids et mieux encore de 0,1 à 3,75 % en poids d'hydroperoxyde, par rapport à la masse de ce premier composant, et dont le deuxième composant contient de 50 à 400 % en moles, de préférence de 75 à 300 % en moles et mieux encore de 100 à 200 % en moles de dérivé de thiourée, par rapport au nombre de moles d'hydroperoxyde présent dans le premier composant.

9. Matériau de dentisterie conforme à l'une des revendications précédentes, qui contient en outre au moins un monomère polymérisable par voie radicalaire, de préférence au moins un (méth)acrylate monofonctionnel ou polyfonctionnel, et mieux encore, au moins un diméthacrylate ou un mélange de monométhacrylate et de diméthacrylate.

10. Matériau de dentisterie conforme à la revendication 9, qui contient, en tant que monomère polymérisable par voie radicalaire, du (méth)acrylate de méthyle, d'éthyle, de 2-hydroxy-éthyle, de butyle, de benzyle, de tétrahydrofurfuryle ou d'isobornyle, du méthacrylate de para-cumyl-phénoxy-éthylèneglycol (CMP-1E), du méthacrylate de 2-(biphényl-2-oxy)-éthyle, du diméthacrylate de bisphénol A, du bis-GMA (produit d'addition à base d'acide méthacrylique et d'éther diglycidylique de bisphénol A), du diméthacrylate de bisphénol A éthoxylé ou propoxylé, du 2-[4-(2-méthacryloyloxy-éthoxy-éthoxy)-phényl]-2-[4-(2-méthacryloyloxy-éthoxy)-phényl]-propane (SR-348c), du 2,2-bis[4-(2-méthacryloxy-propoxy)-phényl]-propane, de l'UDMA (produit d'addition à base de méthacrylate de 2-hydroxy-éthyle et de 2,2,4-triméthyl-hexaméthylène-1,6-diisocyanate), du V-380 (produit d'addition à base d'un mélange de 0,7 mole de méthacrylate de 2-hydroxy-éthyle et de 0,3 mole de méthacrylate de 2-hydroxy-propyle avec 1 mole d'**α**,**α**,**α**',**α**'-tétraméthyl-méta-xylylène-diisocyanate), du diméthacrylate de diéthylèneglycol, de triéthylèneglycol ou de tétra-éthylèneglycol, du triméthacrylate de triméthylolpropane, du tétra-méthacrylate de pentaérythritol, du diméthacrylate et triméthacrylate de glycérol, du diméthacrylate de butane-1,4-diol, du diméthacrylate de décane-1,10-diol (D₃MA), du bis(méthacryloyloxy-méthyl)-tricy-clo[5.2.1.0(2,6)]décane (DCP), un diméthacrylate de polyéthylèneglycol ou de polypropylèneglycol, du diméthacrylate de polyéthylèneglycol-200 ou de polyéthylèneglycol-400 (PEG-200-DMA ou PEG-400-DMA), du diméthacrylate de dodécane-1,12-diol, ou un mélange de ces composés.

11. Matériau de dentisterie conforme à l'une des revendications précédentes, qui contient en outre au moins une charge organique ou inorganique, de préférence un oxyde, tels SiO₂, ZrO₂ et TiO₂, ou un oxyde mixte à base de SiO₂, ZrO₂, ZnO et/ou TiO₂, une charge en nano-particules ou microfine, comme de la silice pyrogénée ou de la silice précipitée, une poudre de verre, comme de la poudre de quartz, de la poudre de vitrocéramique ou de la poudre de verre opaque aux rayons X, de préférence de la poudre de verre aluminosilicate de baryum ou de strontium, une charge opaque aux rayons X, comme du trifluorure d'ytterbium, de l'oxyde de tantale-V, du sulfate de baryum, un oxyde mixte à base de SiO₂ et d'oxyde d'ytterbium-III ou d'oxyde de tantale-V, un prépolymère moulu ou un polymère en perles.

12. Matériau de dentisterie conforme à l'une des revendications précédentes, qui contient
a) 0,005 à 3 % en poids, de préférence 0,025 à 2,5 % en poids, et mieux encore 0,05 à 2,0 % en poids, d'au moins un hydroperoxyde, de préférence, de l'hydroperoxyde de cumène et/ou du propionate de 4-(2-hydroperoxy-prop-2-yl)-phényle,
b) 0,005 à 3,0 % en poids, de préférence 0,015 à 2,125 % en poids, et mieux encore 0,025 à 1,5 % en poids, de dérivés de thiourée,
c) 0,00005 à 0,5 % en poids, de préférence 0,00025 à 0,25 % en poids, et mieux encore 0,00035 à 0,01 % en poids, d'au moins un composé de métal de transition,
d) 5 à 95 % en poids, de préférence 10 à 95 % en poids, et mieux encore 10 à 90 % en poids, d'au moins un monomère polymérisable par voie radicalaire,
e) 0 à 85 % en poids d'au moins une charge,
f) et 0,01 à 5 % en poids, de préférence 0,1 à 3 % en poids, et mieux encore 0,1 à 2 % en poids, d'un ou plusieurs adjuvant(s),
par rapport, dans chaque cas, au poids total de la composition.

13. Matériau de dentisterie conforme à l'une des revendications précédentes, pour utilisation thérapeutique.

14. Utilisation d'un matériau de dentisterie conforme à l'une des revendications 1 à 12 pour la fabrication ou la réparation extra-buccale de restaurations dentaires, telles que prothèses, dents artificielles, incrustations in-lay ou on-lay, couronnes, bridges et prothèses totales.
